(19) Europäisches Patentamt / European Patent Office / Office européen des brevets

(11) **EP 3 805 384 A1**

(12) **EUROPEAN PATENT APPLICATION**

(43) Date of publication:
**14.04.2021 Bulletin 2021/15**

(51) Int Cl.:
***C12N 9/88*** *(2006.01)*  ***C12N 15/09*** *(2006.01)*
***C12P 1/04*** *(2006.01)*

(21) Application number: **20195171.2**

(22) Date of filing: **18.05.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.05.2015 US 201562165690 P**
 **26.05.2015 US 201562166616 P**
 **29.05.2015 US 201562168415 P**

(62) Document number(s) of the earlier application(s) in accordance with Art. 76 EPC:
**16729102.0 / 3 298 137**

(71) Applicant: **DuPont Nutrition Biosciences ApS**
**1411 Copenhagen K (DK)**

(72) Inventors:
• **CRAMER, Jacob Flyvholm**
 **8220 Brabrand (DK)**
• **JENSEN, Lene Bojsen**
 **8270 Højberg (DK)**
• **KELLETT-SMITH, Anja Hemmingsen**
 **1558 Copenhagen V (DK)**
• **LARSEN, Bjarne**
 **8260 Viby J (DK)**

(74) Representative: **D Young & Co LLP**
**120 Holborn**
**London EC1N 2DY (GB)**

Remarks:
•This application was filed on 08.09.2020 as a divisional application to the application mentioned under INID code 62.
•Claims filed after the date of filing of the application / after the date of receipt of the divisional application (Rule 68(4) EPC).

(54) **ALDC PRODUCTION METHODS**

(57) The present disclosure provides methods, compositions, apparatuses and kits comprising ALDC enzymes having a better stability and activity, and which further can be recovered from microorganisms in improved yields.

*FIG. 1*

**Description**

**CROSS-REFERENCE TO RELATED APPLICATIONS**

This applications claims priority to and the benefit of United States provisional patent application numbers 62/165690, filed May 22, 2015; 62/166616, filed May 26, 2015; and 62/168415, filed May 29, 2015; each provisional application titled "ALDC METHODS".

**INCORPORATION BY REFERENCE OF THE SEQUENCE LISTING**

The sequence listing provided in the file named "20160505_NB40737_PCT_SEQS_ST25.txt" with a size of 16,795 bytes which was created on May 5, 2016 and which is filed herewith, is incorporated by reference herein in its entirety.

**BACKGROUND**

Diacetyl is sometimes an unwanted by-product of fermentation processes of carbohydrate containing substances, e.g. wort or grape juice. Formation of diacetyl is most disadvantageous because of its strong and unpleasant smell and in case of beer even small amounts of diacetyl of about 0.10 to 0.15 mg/liter has a negative effect on the flavor and taste of the beer. During the maturation of beer, diacetyl is converted into acetoin by reductases in the yeast cells. Acetoin is with respect to taste and flavor acceptable in beer in much higher concentrations than diacetyl.
Acetolactate decarboxylase (ALDC) can also be used as an enzyme to prevent the formation of diacetyl. $\alpha$-acetolactate can be converted into acetoin by adding an ALDC enzyme during fermentation. However, ALDC can be unstable at fermenting conditions, especially those of fermenting worts with low malt content.
The purpose of the present invention is to provide ALDC enzymes having a better stability and/or activity, and, optionally, the yield of ALDC enzymes which can be recovered from microorganisms is improved.

**SUMMARY OF THE INVENTION**

The present disclosure provides compositions and processes for ALDC enzymes.
Aspects and embodiments of the compositions and methods are set forth in the following separately numbered paragraphs.

1. A method for producing an acetolactate decarboxylase (ALDC) enzyme comprising:

A

i) providing a *Bacillus* host cell comprising a genetic alteration that causes the host cell to produce a decreased amount of an endogenous extracellular serine protease (vpr) and/or a cell wall protease (wprA) when compared to a parental cell, where the host cell is transformed with a nucleic acid encoding a heterologous ALDC enzyme in operable combination with a promoter; and
ii) cultivating the host cell under conditions suitable for the production of the heterologous ALDC enzyme, such that the heterologous ALDC enzyme is produced; or

B

i) providing a *Bacillus* host cell comprising a genetic alteration that causes the host cell to produce a decreased amount of an endogenous extracellular serine protease (vpr) and/or a cell wall protease (wprA) when compared to a parental cell, where the host cell is transformed with a nucleic acid that causes the host cell to overexpress an endogenous nucleic acid sequence encoding an ALDC enzyme when compared to the parental cell; and
ii) cultivating the host cell under conditions suitable for the production of ALDC enzyme, such that ALDC enzyme is produced.

2. The method of paragraph 1, further comprising recovering the produced ALDC enzyme.
3. The method of Paragraph 1 or 2, where the *Bacillus* host cell is B. subtilis.
4. The method of any one of Paragraphs 1 to 3, where the *Bacillus* host cell further lacks an endogenous minor extracellular serine protease enzyme (Epr).

5. The method of any one of Paragraphs 1 to 4, where the *Bacillus* host cell further lacks an endogenous major intracellular serine protease enzyme (IspA), and/or an endogenous bacillopeptidase F enzyme (Bpr).

6. The method of any one of Paragraphs 1 to 5, where the *Bacillus* host cell lacks a neutral metalloprotease enzyme (NprE). The method of paragraph 6, where the *Bacillus* host cell lacks an endogenous neutral metalloprotease enzyme (NprE).

7. The method of any one of Paragraphs 1 to 6, where the host cell further lacks an endogenous serine alkaline protease enzyme (AprE).

8. The method of any one of Paragraphs 1 to 7, where the host cell further lacks an endogenous minor extracellular serine protease enzyme (Vpr).

9. The method of any one of Paragraphs 1 to 8, where the host cell further lacks an endogenous cell wall associated protease enzyme (WprA).

10. The method of any preceding paragraph, where the host further has decreased amounts of one or more additional proteases selected from the group consisting of ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD. The method of any preceding paragraph, where the host further has decreased amounts of one or more additional endogenous proteases selected from the group consisting of ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD.

11. The method of any preceding paragraph, where the genetic alteration comprises a disruption of a gene present in the parental cell.

12. The method of paragraph 11, where the disruption is the result of deletion of all or part of the gene.

13. The method of paragraph 11, where disruption of the gene is the result of deletion of a portion of genomic DNA comprising the gene.

14. The method of any one of paragraphs 11-13, where disruption of the gene is the result of mutagenesis.

15. The method of any one of paragraphs 11-14, where disruption of the gene is performed using site-specific recombination.

16. The method of any one of paragraphs 11-15, where disruption of the gene is performed in combination with introducing a selectable marker at the genetic locus of the gene.

17. The method of any preceding paragraph, where the ALDC enzyme is from *Lactobacillus casei, Brevibacterium acetylicum, Lactococcus lactis, Leuconostoc lactis, Enterobacter aerogenes, Bacillus subtilis, Bacillus brevis, Lactococcus lactis DX,* or *Bacillus licheniformis.*

18. The method of any preceding paragraph, where the ALDC enzyme is from *Bacillus brevis* or *Bacillus licheniformis.*

19. The method of any preceding paragraph, where the ALDC enzyme has an amino acid sequence having at least 80% identity with any one selected from SEQ ID NO: 2, SEQ ID NO: 3, SEQ ID NO: 5, SEQ ID NO: 7, and SEQ ID NO: 8 or any functional fragment thereof.

20. A *Bacillus* host cell comprising a nucleic acid encoding a heterologous ALDC enzyme in operable combination with a promoter, where the host cell comprises a genetic alteration that causes the host cell to produce a decreased amount of an endogenous extracellular serine protease (vpr) and/or a cell wall protease (wprA); or a *Bacillus* host cell where the host cell comprises a genetic alteration that causes the host cell to produce a decreased amount of an endogenous extracellular serine protease (vpr) and/or a cell wall protease (wprA), and where the host cell comprises a nucleic acid that causes the host cell to overexpress an endogenous nucleic acid sequence encoding an ALDC enzyme when compared to the parental cell.

21. The *Bacillus* host cell of Paragraph 20, where the *Bacillus* host cell is *B. subtilis.*

22. The *Bacillus* host cell of Paragraph 20 or Paragraph 21, where the host further has decreased amounts of an endogenous minor extracellular serine protease enzyme (Epr).

23. The *Bacillus* host cell of any one of Paragraphs 20 to 22, where the host further has decreased amounts of an endogenous major intracellular serine protease enzyme (IspA), and/or an endogenous bacillopeptidase F enzyme (Bpr).

24. The *Bacillus* host cell of any one of Paragraphs 20 to 23, where the host further has decreased amounts of a neutral metalloprotease enzyme (NprE). The *Bacillus* host cell of any one of Paragraphs 20 to 23, where the host further has decreased amounts of an endogenous neutral metalloprotease enzyme (NprE).

25. The *Bacillus* host cell of any one of Paragraphs 20 to 24, where the host cell further has decreased amounts of an endogenous serine alkaline protease enzyme (AprE).

26. The *Bacillus* host cell of any one of Paragraphs 20 to 25, where the host cell further has decreased amounts of an endogenous minor extracellular serine protease enzyme (Vpr).

27. The *Bacillus* host cell of any one of Paragraphs 20 to 26, where the host cell further has decreased amounts of an endogenous cell wall associated protease enzyme (WprA).

28. The *Bacillus* host cell of any one of paragraphs 20 to 27, where the host further has decreased amounts of one or more additional proteases selected from the group consisting of ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD. The *Bacillus* host cell of any one of paragraphs 20 to 27, where the host further has decreased amounts of one or more additional endogenous proteases selected from the group consisting of ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD.

29. The *Bacillus* host cell of any one of paragraphs 20 to 28, where the ALDC enzyme is from *Lactobacillus casei, Brevibacterium acetylicum, Lactococcus lactis, Leuconostoc lactis, Enterobacter aerogenes, Bacillus subtilis, Bacillus brevis, Lactococcus lactis DX,* or *Bacillus licheniformis.*

30. The *Bacillus* host cell of any one of paragraphs 20 to 29, where the ALDC enzyme is from *Bacillus brevis* or *Bacillus licheniformis.*

31. The *Bacillus* host cell of any one of paragraphs 20 to 30, where the ALDC enzyme has an amino acid sequence having at least 80% identity with any one selected from SEQ ID NO: 2, SEQ ID NO: 3, SEQ ID NO: 5, SEQ ID NO: 7, and SEQ ID NO: 8 or any functional fragment thereof.

32. A *Bacillus* host cell comprising a nucleic acid encoding a heterologous ALDC enzyme in operable combination with a promoter, where the host cell comprises a genetic alteration that causes the host cell to produce a decreased amount of an endogenous extracellular serine protease and/or a cell wall protease and/or a neutral metalloprotease capable of clipping a sequence from a C-terminus of the ALDC enzyme; or a *Bacillus* host cell where the host cell comprises a genetic alteration that causes the host cell to produce a decreased amount of an endogenous extracellular serine protease and/or a cell wall protease and/or a neutral metalloprotease capable of clipping a sequence from a C-terminus of the ALDC enzyme, and where the host cell comprises a nucleic acid that causes the host cell to overexpress an endogenous nucleic acid sequence encoding an ALDC enzyme when compared to the parental cell.

33. The host cell of paragraph 32, where the ALDC enzyme is *B. brevis* AldB and the sequence from the C-Terminus is QVHQAESERK. The host cell of paragraph 32, where the ALDC enzyme is *B. brevis* AldB and the protease is capable of clipping the sequence QVHQAESERK (SEQ ID NO: 9) from said C-Terminus of said ALDC enzyme.

34. The *Bacillus* host cell of Paragraph 32, where the *Bacillus* host cell is *B. subtilis.*

35. A *Bacillus* host cell comprising a nucleic acid encoding a heterologous ALDC enzyme in operable combination with a promoter, where the host cell comprises a genetic alteration that causes the host cell to produce a decreased amount of a protease when compared to the parental cell, where the protease is capable of cleaving a C-Terminus and/or N-Terminus of the ALDC enzyme; or a *Bacillus* host cell where the host cell comprises a genetic alteration that causes the host cell produce a decreased amount of a protease when compared to the parental cell, where the protease is capable of cleaving a C-Terminus and/or N-Terminus of the ALDC enzyme, and where the host cell comprises a nucleic acid that causes the host cell to overexpress an endogenous nucleic acid sequence encoding an ALDC enzyme when compared to the parental cell.

36. The *Bacillus* host cell of Paragraph 35, where the *Bacillus* host cell is *B. subtilis.*

37. The *Bacillus* host cell of Paragraph 35 or 36, where the protease is capable of cleaving at a corresponding C-terminus position 275 of SEQ ID No:5, SEQ ID No: 2 or SEQ ID NO 7.

38. The *Bacillus* host cell of Paragraph 35 or 36, where the protease is capable of cleaving at a corresponding C-terminus position 276 of SEQ ID No:5, SEQ ID No: 2 or SEQ ID NO 7.

39. The *Bacillus* host cell of Paragraph 35 or 36, where the protease is capable of cleaving at a corresponding N-terminus position 37 of SEQ ID No:5, SEQ ID No: 2 or SEQ ID NO 7.

40. The *Bacillus* host cell of Paragraph 35 or 36, where the protease is capable of cleaving at a corresponding N-terminus position 38 of SEQ ID No:5, SEQ ID No: 2 or SEQ ID NO 7.

41. The *Bacillus* host cell of Paragraph 35 or 36, where the protease is capable of cleaving at a corresponding N-terminus position 39 of SEQ ID No:5, SEQ ID No: 2 or SEQ ID NO 7.

42. The *Bacillus* host cell of Paragraph 35 or 36, where the protease is capable of cleaving at a corresponding N-terminus position 40 of SEQ ID No:5, SEQ ID No: 2 or SEQ ID NO 7.

43. The *Bacillus* host cell of Paragraph 35 or 36, where the protease is capable of cleaving at a corresponding N-terminus position 42 of SEQ ID No:5, SEQ ID No: 2 or SEQ ID NO 7.

44. The *Bacillus* host cell of Paragraph 35 and 36, where the protease is capable of cleaving at a corresponding N-terminus position 43 of SEQ ID No:5, SEQ ID No: 2 or SEQ ID NO 7.

45. The *Bacillus* host cell of Paragraph 35 or 36, where the protease is capable of cleaving at a corresponding N-terminus position 39 of SEQ ID No:5, SEQ ID No: 2 or SEQ ID NO 7.

46. The *Bacillus* host cell of any one of paragraphs 35 to 45, where ALDC enzyme comprises an amino acid sequence that has at least 80% homology to SEQ ID No:5, SEQ ID No: 2 or SEQ ID NO 7.

47. The *Bacillus* host cell of any one of paragraphs 35 to 46, where the ALDC enzyme is *B. brevis* AldB and the sequence from the C-Terminus is QVHQAESERK. The Bacillus host cell of paragraph 35 to 46, wherein said ALDC enzyme is *B. brevis* AldB and the protease is capable of cleaving the sequence QVHQAESERK from said C-Terminus of said ALDC enzyme.

48. The *Bacillus* host cell of any one of paragraphs 35 to 47, where the protease is a neutral metalloprotease.

49. The *Bacillus* host cell of any one of paragraphs 35 to 48, where the protease is thermolysin.

**BRIEF DESCRIPTION OF THE DRAWINGS**

A better understanding of the features and advantages of the present invention will be obtained by reference to the following detailed description that sets forth illustrative embodiments, in which the principles of the invention are utilized, and the accompanying drawings of which:

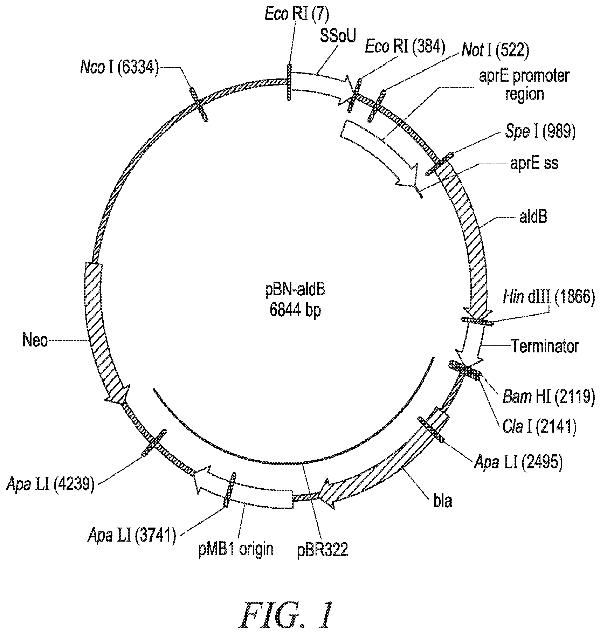

**Figure 1** shows a plasmid map for expression of Acetolactate Decarboxylase, aldB.

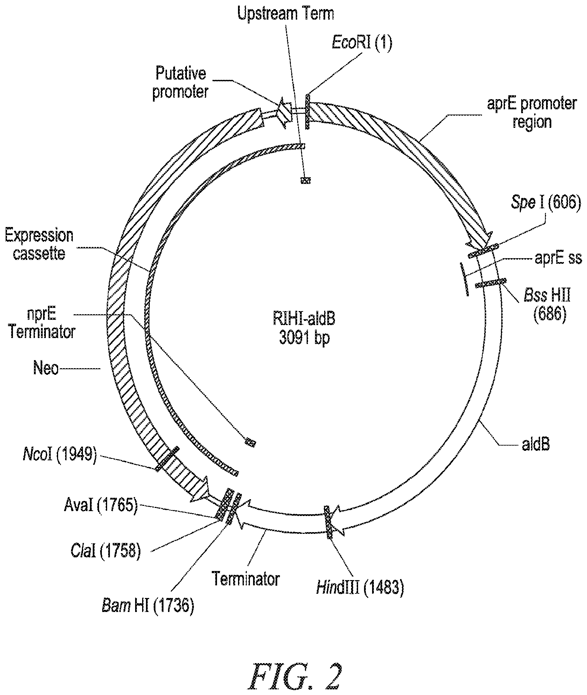

**Figure 2** shows a plasmid map of RIHI-aldB for expression of Acetolactate Decarboxylase, aldB.

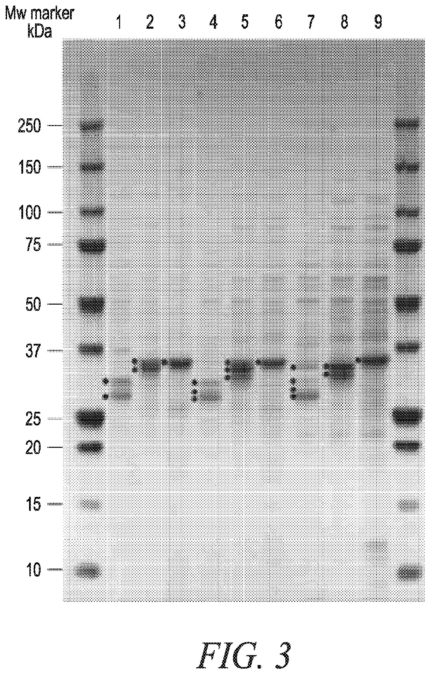

**Figure** 3 shows SDS-PAGE showing truncation variants of aldB expressed in *Bacillus subtilis* strains at various time, Identified AldB variants are marked by a dot. Lane: 1) 24hrs, 2-delete strain, 2) 24hrs, 5-delete strain, 3) 24hrs, 7-delete strain, 4) 48hrs, 2-delete strain, 5) 48hrs, 5-delete strain, 6) 48hrs, 7-delete strain, 7) 72hrs, 2-delete strain, 8) 72hrs, 5-delete strain and 9) 72hrs, 7-delete strain.

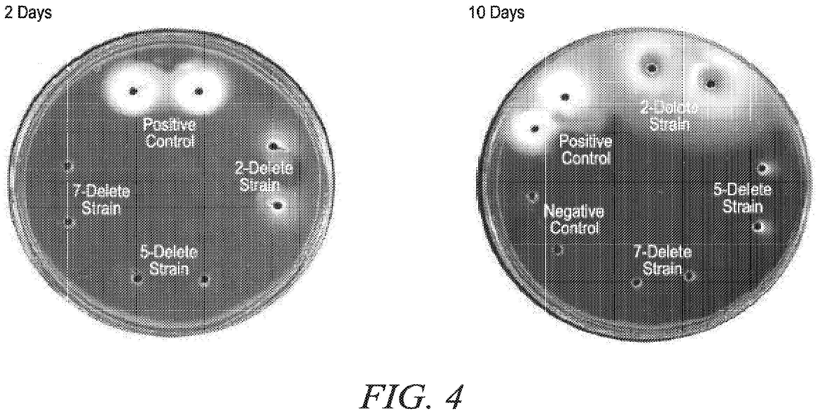

**Figure 4** shows two casein spot plate assay of the 2-, 5-and 7- delete *Bacillus* strains after 2 days (left) and 10 days (right) of incubation at 40°C. Protease activity may be followed by white halo formation. Labels are detailed on the plate; the neutral protease nprE from B. subtilis was used as the positive control and buffer was used as the negative control.

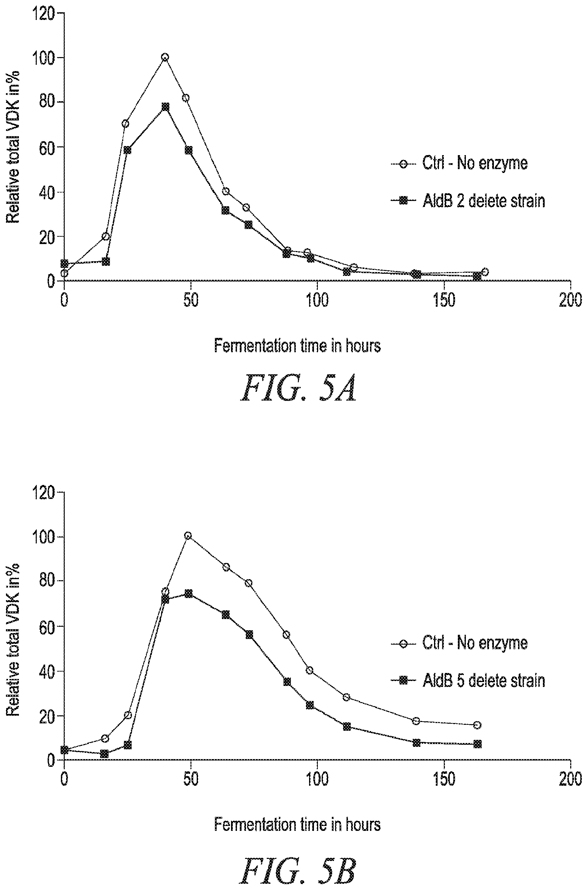

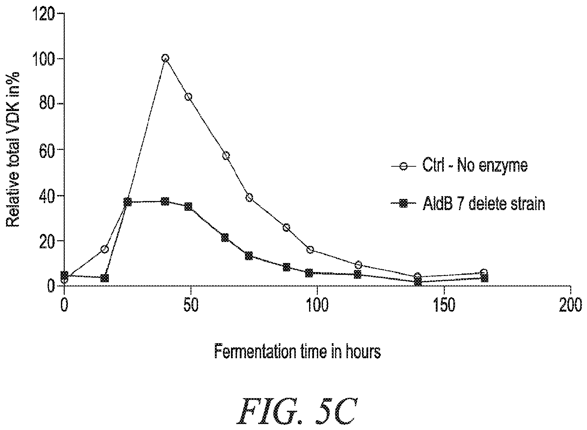

**Figure** 5 shows relative development of vicinal diketones (VDK) during three individual malt-based fermentations in presence or absence of aldB enzyme from various host cells: A) aldB from 2-delete strain, B) aldB from 5-delete strain and C) aldB from 7-delete strain. For comparison, the calculated VDK content (defined as the sum of diacetyl and 2,3 pentanedione) was normalized to the highest value obtained for the control sample without enzyme (100%). All aldB enzymes were dosed with similar ALDC activity 0.04 U/ml wort. The VDK development was followed during the 8 days of fermentation at 14°C.

**DETAILED DESCRIPTION OF THE INVENTION**

The present disclosure provides methods, compositions, apparatuses and kits comprising ALDC enzymes having a better stability and/or activity, and, optionally the yield of ALDC enzymes which can be recovered from microorganisms is improved.

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure which can be had by reference to the specification as a whole. Accordingly, the terms defined immediately below are more fully defined by reference to the specification as a whole.

It must be noted that as used herein and in the appended claims, the singular forms "a", "an", and "the" include plural referents unless the context clearly dictates otherwise. Thus, for example, reference to "a protease" includes a plurality of such enzymes and reference to "the feed" includes reference to one or more feeds and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

All patents and publications referred to herein are incorporated by reference.

**ALDC**

In some aspects the invention provides ALDC enzymes having a better stability and/or activity, and, optionally, the yield of ALDC enzymes which can be recovered from microorganisms is improved.

Acetolactate decarboxylase (ALDC) is an enzyme that belongs to the family of carboxy lyases, which are responsible for cleaving carbon-carbon bonds. Acetolactate decarboxylase catalyzes the conversion of 2-acetolactate (also known as 2-hydroxy-2-methyl-3-oxobutanoate) to 2-acetoin and releases $CO_2$. The terms "ALDC" and "ALDC enzyme" may be used interchangeably herein.

Acetolactate decarboxylase enzymes catalyze the enzymatic reaction belonging to the classification EC 4.1.1.5 (acetolactate decarboxylase activity) and gene ontology (GO) term ID of GO: 0047605. The GO term ID specifies that any protein characterized as having this associated GO term encodes an enzyme with catalytic acetolactate decarboxylase activity.

Various acetolactate decarboxylase genes *(such as alsD or aldB),* which encode acetolactate decarboxylase enzymes, are known in the art. The *alsD* gene, which encodes ALDC enzyme, may be derived or derivable from *Bacillus subtilis.* The *aldB* gene, which encodes ALDC enzyme, may be derived or derivable from *Bacillus brevis.* The *alsD* gene, which encodes ALDC enzyme, may be derived or derivable from *Bacillus licheniformis.* UNIPROT accession number Q65E52.1 is an example of an ALDC enzyme. UNIPROT accession number Q65E52.1 is an example of an ALDC enzyme derived or derivable from *Bacillus licheniformis.* Examples of acetolactate decarboxylase genes include but are not limited to gi|375143627|ref|YP_005006068.1| acetolactate decarboxylase *[Niastella koreensis OR20-10];* gi|361057673|gb|AEV96664.1| acetolactate decarboxylase *[Niastella koreensis OR20-10];* gi|218763415|gb|ACL05881.1| acetolactate decarboxylase *[Desulfatibacillum alkenivorans AK-01];* gi|220909520|ref|YP_002484831.1| acetolactate decarboxylase *[Cyanothece sp. PCC 7425];* gi|218782031|ref|YP_002433349.1| acetolactate decarboxylase *[Desulfatibacillum alkenivorans AK-01];* gi|213693090|ref|YP_002323676.1| acetolactate decarboxylase *[Bifidobacterium longum subsp. infantis* ATCC 15697 = JCM 1222]; gi|189500297|ref|YP_001959767.1| acetolactate decarboxylase *[Chlorobium phaeobacteroides BS1];* gi|189423787|ref|YP_001950964.1| acetolactate decarboxylase *[Geobacter lovleyi SZ];* gi|172058271|ref|YP_001814731.1| acetolactate decarboxylase *[Exiguobacterium sibiricum 255-15];* gi|163938775|ref|YP_001643659.1| acetolactate decarboxylase *[Bacillus weihenstephanensis KBAB4];* gi|158522304|ref|YP_001530174.1| acetolactate decarboxylase *[Desulfococcus oleovorans Hxd3];* gi|57371670|ref|YP_001479659.1| acetolactate decarboxylase *[Serratiaproteamaculans 568];* gi|150395111|ref|YP_001317786.1| acetolactate decarboxylase [Staphylococcus aureus subsp. aureus JH1]; gi|150394715|ref|YP_001317390.1| acetolactate decarboxylase *[Staphylococcus aureus subsp. aureus JH1];* gi|146311679|ref|YP_001176753.1| acetolactate decarboxylase *[Enterobacter sp.* 638]; gi|109900061|ref|YP_663316.1| acetolactate decarboxylase *[Pseudoalteromonas atlantica T6c];* gi|219866131|gb|ACL46470.1| acetolactate decarboxylase *[Cyanothece sp.* PCC 7425]; gi|213524551|gb|ACJ53298.1| acetolactate decarboxylase *[Bifidobacterium longum* subsp. *infantis* ATCC 15697 = JCM 1222]; gi|189420046|gb|ACD94444.1| acetolactate decarboxylase *[Geobacter lovleyi SZ];* gi|158511130|gb|ABW68097.1| acetolactate decarboxylase *[Desulfococcus oleovorans Hxd3];* gi|157323434|gb|ABV42531.1| acetolactate decarboxylase *[Serratia proteamaculans 568];* gi|145318555|gb|ABP60702.1| acetolactate decarboxylase *[Enterobacter sp.* 638*];* gi|1499475631gb|ABR53499.1| acetolactate decarboxylase *[Staphylococcus aureus subsp.aureus JH1];* gi|149947167|gb|ABR53103.1| acetolactate decarboxylase *[Staphylococcusaureus subsp. aureus JH1];* gi|163860972|gb|ABY42031.1| Acetolactate decarboxylase *[Bacillus weihenstephanensis KBAB4];* gi|109702342|gb|ABG42262.1| Acetolactate decarboxylase *[Pseudoalteromonas atlantica T6c];* gi|189495738|gb|ACE04286.1| acetolactate decarboxylase *[Chlorobium phaeobacteroides BS1];* gi|171990792|gb|ACB61714.1| acetolactate decarboxylase *[Exiguobacterium sibiricum 255-15];* gi|223932563|ref|ZP_03624564.1 acetolactate decarboxylase *[Streptococcus suis 89/1591];* gi|194467531|ref|ZP_03073518.1| acetolactate decarboxylase *[Lactobacillus reuteri 100-23];* gi|223898834|gb|EEF65194.1| acetolactate decarboxylase *[Streptococcus suis 89/1591];* gi|194454567|gb|EDX43464.1| acetolactate decarboxylase *[Lactobacillus reuteri 100-23];* gi|384267135|ref|YP_005422842.1| acetolactate decarboxylase *[Bacillus amyloliquefaciens subsp. plantarum* YAU B9601-Y2]; gi|375364037|ref|YP_005132076.1| acetolactate decarboxylase *[Bacillus amyloliquefaciens subsp. plantarum* CAU B946]; gi|34079323|ref|YP_004758694.1| acetolactate decarboxylase *[Corynebacterium variabile* DSM 44702]; gi|336325119|ref|YP_004605085.1| acetolactate decarboxylase *[Corynebacterium resistens* DSM 45100]; gi|148269032|ref|YP_001247975.1| acetolactate decarboxylase *[Staphylococcus aureus subsp. aureus JH9];* gi|148268650|ref|YP_001247593.1| acetolactate decarboxylase *[Staphylococcus aureus subsp. aureus JH9];* gi|1485433721|ref|YP_001270742.1| acetolactate decarboxylase *[Lactobacillus reuteri* DSM 20016]; gi|380500488|emb|CCG51526.1| acetolactate decarboxylase *[Bacillus amyloliquefaciens subsp. plantarum* YAU B9601-Y2]; gi|371570031|emb|CCF06881.1| acetolactate decarboxylase *[Bacillus amyloliquefaciens subsp. plantarum* CAU B946]; gi|340533141|gb|AEK35621.1| acetolactate decarboxylase *[Corynebacterium variabile* DSM 44702]; gi|336101101|gb|AE108921.1| acetolactate decarboxylase *[Corynebacterium resistens* DSM 45100]; gi|148530406|gb|ABQ82405.1| acetolactate decarboxylase *[Lactobacillus reuteri* DSM 20016]; gi|147742101|gb|ABQ50399.1| acetolactate decarboxylase *[Staphylococcus aureus subsp. aureus JH9];* gi|147741719|gb|ABQ50017.1| acetolactate decarboxylase *[Staphylococcus aureus subsp. aureus JH9];*

gi|392529510|ref|ZP_10276647.1| acetolactate decarboxylase *[Carnobacterium maltaromaticum* ATCC 35586]; gi|366054074|ref|ZP_09451796.1| acetolactate decarboxylase *[Lactobacillus suebicus* KCTC 3549]; gi|339624147|ref|ZP_08659936.1| acetolactate decarboxylase *[Fructobacillus jructosus* KCTC 3544]; and gi|336393727|ref|ZP_08575126.1| acetolactate decarboxylase *[Lactobacillus coryniformis subsp. torquens* KCTC 3535]. UNIPROT Accession No. P23616.1 (Diderichsen et al., J Bacteriol. (1990) 172(8): 4315) is an example of an ALDC enzyme. UNIPROT accession number P23616.1 is an example of an ALDC enzyme derived or derivable from *Bacillus brevis.* Each sequence associated with the foregoing accession numbers is incorporated herein by reference.

In some embodiments, the invention relates to ALDC enzymes from *Lactobacillus casei* (Godtfredsen 1984), *Brevibacterium acetylicum* (Oshiro, 1989), *Lactococcus lactis* (Vincent Phalip 1994), *Leuconostoc lactis* (O sulivan, 2001), *Enterobacter aerogenes* (Blomquist, 1993), *Bacillus subtilis* (Renna, 1993), *Bacillus brevis* (Svendsen, 1989) and *Lactococcus lactis DX* (Yuxing, 2014). In some embodiments, the ALDC enzyme is from *Lactobacillus casei, Brevibacterium acetylicum, Lactococcus lactis, Leuconostoc lactis, Enterobacter aerogenes, Bacillus subtilis, Bacillus brevis, Lactococcus lactis DX,* or *Bacillus licheniformis.* As used herein, the term "ALDC enzyme is from" refers to the ALDC enzyme being derived or derivable from.

It is to be understood that any suitable ALDC enzymes, i.e. ALDC produced from any microorganism which activity is dependent on metal ions, can be used according to the invention. In some embodiments, the ALDC used in the methods and compositions described herein is an ALDC from *Bacillus brevis* or *Bacillus licheniformis.*

The ALDC activity of the enzyme composition according to the invention is measured by the ALDC assays as described herein or any suitable assay known in the art. The standard assay is carried out at pH 6.0, and it can be performed at different pH values and temperatures for the additional characterization and specification of enzymes.

One unit of ALDC activity is defined as the amount of enzyme which produces 1 $\mu$mole acetoin per minute under the conditions of the assay (e.g., pH 6.0 (or as specified) and 30 °C).

In some embodiments, the ALDC enzyme comprises an amino acid sequence having at least 70%, 75%, 80%, 85%, 90%, 95%, 97%, 98%, 99% or 100% identity with SEQ ID NO: 2, SEQ ID NO: 3, SEQ ID NO: 5, SEQ ID NO: 7, SEQ ID NO: 8 or any functional fragment thereof. One aspect of the invention relates to an enzyme exhibiting ALDC activity, which enzyme comprises an amino acid sequence having at least 80% identity with any one selected from SEQ ID NO: 2, SEQ ID NO: 3, SEQ ID NO: 5, SEQ ID NO: 7, and SEQ ID NO: 8, or any functional fragment thereof. In some embodiments, the ALDC enzyme is encoded by a nucleic acid sequence having at least 70%, 75%, 80%, 85%, 90%, 95%, 97%, 98%, 99% or 100% identity with SEQ ID NO: 1, SEQ ID NO: 4, SEQ ID NO: 6, or any functional fragment thereof. The terms "nucleic acid", "nucleic acid sequence" and "nucleotide sequence" used herein are interchangeable.

In some embodiments, the enzyme has a temperature optimum in the range of 5-80 °C, such as in the range of 5-40°C or 15-80°C, such as in the range 20-80 °C, such as in the range 5-15°C, 10-40°C, 10-50°C, 15-20°C, 45-65 °C, 50-65 °C, 55-65 °C or 60-80°C. In some embodiments, the enzyme has a temperature optimum in the range of 45-65 °C. In some embodiments, the enzyme has a temperature optimum of about 60°C.

In some embodiments, the enzyme has a total number of amino acids of less than 350, such as less than 340, such as less than 330, such as less than 320, such as less than 310, such as less than 300 amino acids, such as in the range of 200 to 350, such as in the range of 220 to 345 amino acids.

In some embodiments, the amino acid sequence of the enzyme has at least one, two, three, four, five, six, seven, eight, nine or ten amino acid substitutions as compared to any one amino acid sequence selected from SEQ ID NO: 2, SEQ ID NO: 3, SEQ ID NO: 5, SEQ ID NO: 7, and SEQ ID NO: 8, or any functional fragment thereof.

In some embodiments, the amino acid sequence of the enzyme has a maximum of one, two, three, four, five, six, seven, eight, nine or ten amino acid substitutions compared to any one amino acid sequence selected from SEQ ID NO: 2, SEQ ID NO: 3, SEQ ID NO: 5, SEQ ID NO: 7, and SEQ ID NO: 8, or any functional fragment thereof.

In some embodiments, the enzyme comprises the amino acid sequence identified by any one of SEQ ID NO: 2, SEQ ID NO: 3, SEQ ID NO: 5, SEQ ID NO: 7, SEQ ID NO: 8, or any functional fragment thereof.

In some embodiments, the enzyme consists of the amino acid sequence identified by any one of SEQ ID NO: 2, SEQ ID NO: 3, SEQ ID NO: 5, SEQ ID NO: 7, SEQ ID NO: 8, or any functional fragment thereof.

In some embodiments the ALDC compositions, media and methods according to the invention comprise any one or more further enzyme. In some embodiments the one or more further enzyme is selected from list consisting of acetolactate reductoisomerases, acetolactate isomerases, amylase, glucoamylase, hemicellulase, cellulase, glucanase, pullulanase, isoamylase, endo-glucanase and related beta-glucan hydrolytic accessory enzymes, xylanase, xylanase accessory enzymes (for example, arabinofuranosidase, ferulic acid esterase, and xylan acetyl esterase), beta-glucosidase and protease.

In some embodiments the compositions, media and methods according to the invention comprise an enzyme exhibiting ALDC activity, wherein the activity of said ALDC enzyme is in the range of 950 to 2500 Units per mg of protein. In some embodiments the compositions, media and methods according to the invention comprise an enzyme exhibiting ALDC activity, wherein the activity of said ALDC enzyme is in the range of 1000 to 2500 Units per mg of protein. In some embodiments the compositions, media and methods according to the invention comprise an enzyme exhibiting ALDC activity, wherein the activity of said ALDC enzyme is in the range of 1500 to 2500 Units per mg of protein. In some embodiments, the enzyme exhibiting ALDC activity is an enzyme comprising an amino acid sequence having at least 80% identity with any one selected from SEQ ID NO: 2, SEQ ID NO: 3, SEQ ID NO: 5, SEQ ID NO: 7, and SEQ ID NO: 8 or any functional fragment thereof. In some embodiments, the enzyme exhibiting ALDC activity is encoded by a nucleic acid sequence having at least 80% identity with SEQ ID NO: 1, SEQ ID NO: 4, SEQ ID NO: 6 or any functional fragment thereof.

**Host**

In one aspect the invention provides host cells having one or more genetic alterations that give increased ALDC enzyme yields and/or produce ALDC enzymes with increased stability and/or activity. The terms "host cell comprising a genetic alteration", "host cell comprises a genetic alteration", "variant host cell", "variant strain", "variant", "host cell" and "microorganism" may be used interchangeably herein. As used herein the term "genetic alteration" refers to a host cell which has at least one genetic alteration when compared to the parental host cell; typically, the genetic alteration is a modification to the genome of the host cell. The genetic alteration in the host cell may be the result of a spontaneous mutation and/or genetic engineering (such as transformation of the cell with a vector or removal of specific genes from the genome). Examples of genetic alterations include genetic alterations that cause the cells of the variant strain to produce a decreased amount of at least one endogenous protease when compared to the parental cell. In one embodiment, the variant strain is further modified to comprise a heterologous nucleic acid sequence encoding an ALDC enzyme when compared to the parental strain. In one embodiment, the parental strain comprises a heterologous nucleic acid sequence encoding an ALDC enzyme. In one embodiment, the variant strain has been modified to overexpress an endogenous nucleic acid sequence encoding an ALDC enzyme when compared to the parental strain. In one embodiment, the variant strain is transformed with a nucleic acid that causes the host cell to overexpress an endogenous nucleic acid sequence encoding an ALDC enzyme when compared to the parental strain. As used herein, the terms "overexpressing", "overexpression" and "overexpress" refer an increase in the expression of the nucleic acid sequence encoding an ALDC enzyme in the variant strain when compared to the expression of the nucleic acid sequence encoding an ALDC enzyme in the parental strain when cultured under the same conditions permitting expression of the nucleic acid sequence encoding ALDC. For example, the expression of the nucleic acid sequence encoding an ALDC enzyme by the variant strain is at least 30%, 40%, 50%, 60%, 70%, 80% or 90% higher than the expression of the nucleic acid sequence encoding an ALDC enzyme by the parental strain. Without wishing to be bound by theory, the overexpression of an endogenous nucleic acid sequence encoding an ALDC enzyme in a host cell causes the increased production of ALDC enzyme in the host cell when compared to the production of ALDC enzyme in the parental strain. Host cells may be identified by screening for cells which have reduced protease activity and/or protease functionality when compared to the parental cell. The genetic alteration may disrupt, for example, one or more genes encoding a protease. Examples of proteases encoded by such genes include: wprA, vpr, epr, ispA, bpr, nprE, aprE, ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD. The genetic alteration may disrupt, for example, a regulatory sequence (e.g. a promoter) of a gene encoding a protease or a nucleic acid sequence capable of regulating the expression a protease. Without wishing to be bound by theory, the disruption may cause the deletion of all or part of the gene and/or regulatory sequence. The genetic alteration may, for example, result from the introduction into the host cell of a nucleic acid sequence encoding an antisense nucleic acid sequence capable of binding to a nucleic acid sequence encoding a protease or a nucleic acid sequence capable of regulating the expression of a protease. The genetic alteration may be the deletion of all or part of, for example, one or more genes encoding a protease (such as WprA, Vpr, Epr, IspA, Bpr, NprE, AprE, ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD) or a nucleic acid sequence capable of regulating the expression of a gene encoding a protease. The genetic alteration may be the deletion of, for example, a regulatory sequence (e.g. a promoter) of a gene encoding a protease or a nucleic acid sequence capable of regulating the expression a protease. The genetic alteration may be the deletion of a portion of genomic DNA comprising, for example, a gene encoding a protease or a nucleic acid sequence capable of regulating the expression of a gene encoding a protease. The term "improved" and the term "increased" in connection to the yield of ALDC enzyme refer to an increase in the amount of protein having ALDC activity which is produced (e.g. recovered) when a host cell having a genetic alteration is cultured compared to the amount of protein having ALDC activity which is produced by the parental host cell when cultured under the same conditions (e.g. the same time and temperature). The terms "better stability" and "increased stability" as used herein refer to an ALDC enzyme produced by a host cell having a genetic alteration maintaining the ALDC activity for a longer period of time when compared to the ALDC activity of the ALDC enzyme produced by the parental strain when cultured under the same conditions (e.g. the same temperature). The terms "better activity" and "increased activity" as used herein refer to an ALDC enzyme produced by a host cell having a genetic alteration having an ALDC activity which is increased when compared to the ALDC activity of the ALDC enzyme produced by the parental strain when cultured under the same conditions (e.g. the same time and temperature). As used herein, the term "decreased amount" in connection to a protease refers to the amount of the protease which is produced by a host cell having a genetic alteration being decreased when compared to the amount of the protease produced by the parental strain when cultured under the same conditions. As used herein, the term "decreased amount" in connection to a functional protease refers to the amount and/or the activity of the protease which is produced by a host cell having a genetic alteration being decreased when compared to the amount and/or activity of the protease produced by the parental strain when cultured under the same conditions. The activity of proteases is measured by the protease assays as described herein (e.g. casein spot plate assay) or any suitable assay known in the art. In some embodiments, the host cell according to the present invention has no clear halo formation after 2, 5 or 10 days of incubation using the Casein assay described in the Examples.

In some embodiments, the ALDC enzyme is a ALDC enzyme from *Lactobacillus casei* (Godtfredsen 1984), *Brevibacterium acetylicum* (Oshiro, 1989), *Lactococcus lactis* (Vincent Phalip 1994), *Leuconostoc lactis* (O sulivan, 2001), *Enterobacter aerogenes* (Blomquist, 1993), *Bacillus subtilis* (Renna, 1993), *Bacillus brevis* (Svendsen, 1989) and *Lactococcus lactis DX* (Yuxing, 2014).

In some embodiments, the ALDC enzyme is from *Lactobacillus casei, Brevibacterium acetylicum, Lactococcus lactis, Leuconostoc lactis, Enterobacter aerogenes, Bacillus subtilis, Bacillus brevis, Lactococcus lactis DX,* or *Bacillus licheniformis.* In some embodiments, the ALDC enzyme is an ALDC from *Bacillus brevis* or *Bacillus licheniformis.*

In one embodiment, a variant host cell derived or derivable from a parental cell is provided. In one aspect, the variant host cell comprises one or more genetic alterations that causes cells of the variant strain to produce a decreased amount of one or more proteases when compared to the parental cell; preferably said protease is selected from the group consisting of WprA, Vpr, Epr, IspA, Bpr, NprE, AprE, ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD. Such a host cell may be referred to herein as "protease deficient" or a "protease minus strain". In one aspect, the variant host cell comprises a nucleic acid sequence encoding a heterologous ALDC enzyme. In one aspect, the variant host cell comprises one or more genetic alterations that causes the host cell to overexpress an endogenous nucleic acid sequence encoding an ALDC enzyme when compared to the parental cell. In one aspect, the variant host cell comprises a nucleic acid that causes the host cell to overexpress an endogenous nucleic acid sequence encoding an ALDC enzyme when compared to the parental cell. In one aspect, a variant host cell derived from a parental cell is provided, the variant host cell comprises a genetic alteration that causes cells of the variant strain to produce a decreased amount of functional protease when compared to the parental cell, wherein the protease is capable of cleaving a C-Terminus and/or N-Terminus of an ALDC enzyme. The term "the protease is capable of cleaving a C-Terminus and/or N-Terminus of the ALDC enzyme" as used herein refers to the protease cleaving the ALDC enzyme at the C-Terminus and/or the N-terminus. For example, the protease may cleave the ALDC enzyme at an amino acid position which corresponds to amino acid position 275 or 276 of SEQ ID NO 5, SEQ ID No 2 or SEQ ID No 7; in addition or alternatively, the protease may cleave the ALDC enzyme at an amino acid position which corresponds to amino acid position 37, 38, 39, 40, 42 or 43 of SEQ ID NO 5, SEQ ID No 2 or SEQ ID No 7. Amino acids at position 275 or 276 of SEQ ID NO 5, SEQ ID No 2 or SEQ ID No 7 are at the C-terminus of ALDC. Amino acids at position 37, 38, 39, 40, 42 or 43 of SEQ ID NO 5, SEQ ID No 2 or SEQ ID No 7 are at the N-terminus of ALDC. The protease referred to herein may be capable of cleaving the sequence QVHQAESERK from the C-Terminus of an ALDC enzyme such as an ALDC having at least 80% identity to the sequence shown as SEQ ID No:5 or SEQ ID No:2 or SEQ ID No:7. The terms "clipping" and "cleaving" may be used interchangeably herein. In some embodiments, the protease is capable of cleaving at corresponding C-terminus position 275 of SEQ ID No:5 or SEQ ID No:2 or SEQ ID No:7. In some embodiments, the protease is capable of cleaving at corresponding C-terminus position 276 of SEQ ID No:5 or SEQ ID No:2 or SEQ ID No:7. In some embodiments, the protease is capable of cleaving at corresponding N-terminus position 37 of SEQ ID No:5 or SEQ ID No:2 or SEQ ID No:7. In some embodiments, the protease is capable of cleaving at corresponding N-terminus position 38 of SEQ ID No:5 or SEQ ID No:2 or SEQ ID No:7. In some embodiments, the protease is capable of cleaving at corresponding N-terminus position 39 of SEQ ID No:5 or SEQ ID No:2 or SEQ ID No:7. In some embodiments, the protease is capable of cleaving at corresponding N-terminus position 40 of SEQ ID No:5 or SEQ ID No:2 or SEQ ID No:7. In some embodiments, the protease is capable of cleaving at corresponding N-terminus position 42 of SEQ ID No:5 or SEQ ID No:2 or SEQ ID No:7. In some embodiments, the protease is capable of cleaving at corresponding N-terminus position 43 of SEQ ID No:5 or SEQ ID No:2 or SEQ ID No:7. In some embodiments, the protease is capable of cleaving at corresponding N-terminus position 39 of SEQ ID No:5 or SEQ ID No:2 or SEQ ID No:7. In some embodiments, the protease is a neutral metalloprotease. NprE is an example of a neutral metalloprotease. In some embodiments, the protease is thermolysin. Thermolysin may be used in gluten hydrolysis and/or in grain processing.

In some embodiments, the host cell is a bacterial host such as *Bacillus.* In some embodiments, the host cell is *Bacillus.* In some embodiments, the *Bacillus* is *B. subtilis.* In some embodiments the ALDC enzyme is produced by cultivation of a *Bacillus subtilis.*

In some embodiments, the invention provides a variant host cell comprising a genetic alteration that causes cells of the variant strain to produce a decreased amount of a functional endogenous cell wall associated protease enzyme (WprA) when compared to the parental cell, wherein the host cell is transformed with a nucleic acid encoding a heterologous ALDC enzyme in operable combination with a promoter. In some embodiments, the invention provides a variant host cell comprising a genetic alteration that causes cells of the variant strain to produce a decreased amount of a functional endogenous cell wall associated protease enzyme (WprA) when compared to the parental cell, wherein the host cell is transformed with a nucleic acid that causes the host cell to overexpress an endogenous nucleic acid sequence encoding an ALDC enzyme when compared to the parental cell. As used herein, the terms "cell wall associated protease" and "WprA" may be interchangeable with the terms "cell wall protease" and "wprA". In some embodiments, the host cell is *Bacillus.* In some embodiments, the *Bacillus* is *B. subtilis.*

In some embodiments, the genetic alteration comprises a disruption of the wprA gene present in the parental strain. In some embodiments, disruption of the wprA gene is the result of deletion of all or part of the wprA gene. In some embodiments, disruption of the wprA gene is the result of deletion of a portion of genomic DNA comprising the wprA gene. In some embodiments, disruption of the wprA gene is the result of mutagenesis of the wprA gene.

In some embodiments, disruption of the wprA gene is performed using site-specific recombination. In some embodiments, disruption of the wprA gene is performed in combination with introducing a selectable marker(such as e.g. a spectinomycin resistance gene) at the genetic locus of the wprA gene.

In some embodiments, the variant strain does not produce functional wprA protein. In some embodiments, the variant strain does not produce wprA protein.

In some embodiments, the decreased amount of the WprA protein is achieved by any suitable method known in the art, e.g. anti-sense RNA.

In some embodiments, the invention provides a variant host cell comprising a genetic alteration that causes cells of the variant strain to produce a decreased amount of a functional extracellular serine protease enzyme (Vpr) when compared to the parental cell, wherein the host cell is transformed with a nucleic acid encoding a heterologous ALDC enzyme in operable combination with a promoter. In some embodiments, the invention provides a variant host cell comprising a genetic alteration that causes cells of the variant strain to produce a decreased amount of a functional extracellular serine protease enzyme (Vpr) when compared to the parental cell, wherein the host cell is transformed with a nucleic acid that causes the host cell to overexpress an endogenous nucleic acid sequence encoding an ALDC enzyme when compared to the parental cell. As used herein, the terms "extracellular serine protease enzyme" and "vpr" may be interchangeable with the terms "minor extracellular serine protease enzyme", "Vpr", "epr" and "Epr". In some embodiments, the host cell is *Bacillus.* In some embodiments, the *Bacillus* is *B. subtilis.*

In some embodiments, the genetic alteration comprises a disruption of the vpr gene present in the parental strain. In some embodiments, disruption of the vpr gene is the result of deletion of all or part of the vpr gene. In some embodiments, disruption of the vpr gene is the result of deletion of a portion of genomic DNA comprising the vpr gene. In some embodiments, disruption of the vpr gene is the result of mutagenesis of the vpr gene.

In some embodiments, disruption of the vpr gene is performed using site-specific recombination. In some embodiments, disruption of the vpr gene is performed in combination with introducing a selectable marker (such as e.g. a Kanamycin resistance gene) at the genetic locus of the vpr gene.

In some embodiments, the variant strain does not produce functional vpr protein. In some embodiments, the variant strain does not produce vpr protein.

In some embodiments, the decreased amount of the Vpr protein is achieved by any suitable method known in the art, e.g. anti-sense RNA.

In some embodiments, the invention provides a variant host cell comprising a genetic alteration that causes cells of the variant strain to produce a decreased amount of both a functional extracellular serine protease enzyme (Vpr) and of a functional endogenous cell wall associated protease enzyme (WprA) when compared to the parental cell, wherein the host cell is transformed with a nucleic acid encoding a heterologous ALDC enzyme in operable combination with a promoter. In some embodiments, the invention provides a variant host cell comprising a genetic alteration that causes cells of the variant strain to produce a decreased amount of both a functional extracellular serine protease enzyme (Vpr) and of a functional endogenous cell wall associated protease enzyme (WprA) when compared to the parental cell, wherein the host cell is transformed with a nucleic acid that causes the host cell to overexpress an endogenous nucleic acid sequence encoding an ALDC enzyme when compared to the parental cell. In some embodiments, the host cell is *Bacillus.* In some embodiments, the *Bacillus* is *B. subtilis.* In some embodiments, the decreased amount of the proteins is achieved by any of the methods described herein or by any suitable method known in the art, e.g. anti-sense RNA. In some embodiments, the variant strain does not produce functional Vpr and WprA protein. In some embodiments, the variant strain does not produce Vpr and WprA protein.

In some embodiments, the host cell further has a genetic alteration that causes cells of the variant strain to produce a decreased amount of both an endogenous serine alkaline protease enzyme (AprE), and an endogenous extracellular neutral metalloprotease enzyme (NprE), wherein the host cell is transformed with a nucleic acid encoding a heterologous ALDC enzyme in operable combination with a promoter. In some embodiments, the host cell further has a genetic alteration that causes cells of the variant strain to produce a decreased amount of both an endogenous serine alkaline protease enzyme (AprE), and an endogenous extracellular neutral metalloprotease enzyme (NprE), wherein the host cell is transformed with a nucleic acid that causes the host cell to overexpress an endogenous nucleic acid sequence encoding an ALDC enzyme when compared to the parental cell. In some embodiments, the host cell is *Bacillus.* In some embodiments, the *Bacillus* is *B. subtilis.* In some embodiments, the decreased amount of the proteins is achieved by any of the methods described herein or by any suitable method known in the art, e.g. anti-sense RNA. In some embodiments, the variant strain does not produce functional NprE and AprE protein. In some embodiments, the variant strain does not produce NprE and AprE protein.

In some embodiments, the host cell further has decreased amount of an endogenous minor extracellular serine protease enzyme (Epr). As used herein, the term "lacks" is interchangeable with the term "decreased amount". In additional embodiments, the host cell further lacks one or both of an endogenous major intracellular serine protease enzyme (IspA), and an endogenous bacillopeptidase F enzyme (Bpr). In some embodiments, the host cell is *Bacillus.* In some embodiments, the *Bacillus* is *B. subtilis.* In some embodiments, the decreased amount of the proteins is achieved by any of the methods described herein or by any suitable method known in the art, e.g. anti-sense RNA. In some embodiments, the variant strain does not produce functional Epr, IspA and Bpr protein. In some embodiments, the variant strain does not produce Epr, IspA and Bpr protein.

In some embodiments, the present invention provides a host cell having decreased amounts of an endogenous serine alkaline protease enzyme (AprE), an endogenous extracellular neutral metalloprotease enzyme (NprE), an endogenous minor extracellular serine protease enzyme (Vpr), an endogenous minor extracellular serine protease enzyme (Epr), an endogenous major intracellular serine protease enzyme (IspA), and an endogenous bacillopeptidase F enzyme (Bpr). In some embodiments, the host cell is *Bacillus.* In some embodiments, the *Bacillus* is *B. subtilis.* In some embodiments, the decreased amount of the proteins is achieved by any of the methods described herein or by any suitable method known in the art, e.g. anti-sense RNA. In some embodiments, the variant strain does not produce functional AprE, NprE, Vpr, Epr, IspA and Bpr protein. In some embodiments, the variant strain does not produce AprE, NprE, Vpr, Epr, IspA and Bpr protein.

In some embodiments, the present invention provides a host cell having decreased amounts of an endogenous serine alkaline protease enzyme (AprE), an endogenous extracellular neutral metalloprotease enzyme (NprE), an endogenous cell wall associated protease enzyme (WprA), an endogenous minor extracellular serine protease enzyme (Epr), an endogenous major intracellular serine protease enzyme (IspA), and an endogenous bacillopeptidase F enzyme (Bpr). In some embodiments, the host cell is *Bacillus.* In some embodiments, the *Bacillus* is *B. subtilis.* In some embodiments, the decreased amount of the proteins is achieved by any of the methods described herein or by any suitable method known in the art, e.g. anti-sense RNA. In some embodiments, the variant strain does not produce functional AprE, NprE, WprA, Epr, IspA and Bpr protein. In some embodiments, the variant strain does not produce AprE, NprE, WprA, Epr, IspA and Bpr protein.

In some embodiments the present invention provides a host cell having decreased amounts of an endogenous serine alkaline protease enzyme (AprE), an endogenous extracellular neutral metalloprotease enzyme (NprE), an endogenous minor extracellular serine protease enzyme (Vpr), an endogenous minor extracellular serine protease enzyme (Epr), an endogenous major intracellular serine protease enzyme (IspA), an endogenous bacillopeptidase F enzyme (Bpr), and an endogenous cell wall associated protease enzyme (WprA). In some embodiments, the host cell is *Bacillus.* In some embodiments, the *Bacillus* is *B. subtilis.* In some embodiments, the *Bacillus* is *B. subtilis.* In some embodiments, the decreased amount of the proteins is achieved by any of the methods described herein or by any suitable method known in the art, e.g. anti-sense RNA. In some embodiments, the variant strain does not produce functional AprE, NprE, WprA, Vpr, Epr, IspA and Bpr protein. In some embodiments, the variant strain does not produce AprE, NprE, WprA, Vpr, Epr, IspA and Bpr protein.

In additional embodiments, the host cell further has decreased amounts of one or more additional proteases, such ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, ywaD, or other proteases known to those of skill in the art. In some embodiments, the host cell is *Bacillus.* In some embodiments, the *Bacillus* is *B. subtilis.*

Moreover the present invention provides compositions comprising an ALDC enzyme produced by cultivation of a host as described herein, wherein the composition has reduced amounts of *Bacillus* protease activity. In some embodiments, the composition comprise less than 0.50 U/ml protease activity, preferably less than 0.05 U/ml protease activity, and more preferably less than 0.005 U/ml protease activity. In some embodiments, the composition has no clear halo formation after 10 days of incubation using the Casein assay described in the Examples. In some embodiments, the present invention provides compositions comprising an ALDC enzyme produced by cultivation of a host as described herein, wherein the composition is essentially devoid of *Bacillus* serine protease enzyme (AprE) contamination. In some preferred embodiments, the AprE contamination comprises less than about 1% by weight as compared to the ALDC enzyme thereof. In some preferred embodiments, the AprE contamination comprises less than 0.50 U/ml serine protease activity, preferably less than 0.05 U/ml serine protease activity, and more preferably less than 0.005 U/ml serine protease activity. In some particularly preferred embodiments, the ALDC compositions further comprise at least one additional enzyme or enzyme derivative selected from acetolactate reductoisomerases, acetolactate isomerases, amylase, glucoamylase, hemicellulase, cellulase, glucanase, pullulanase, isoamylase, endo-glucanase and related beta-glucan hydrolytic accessory enzymes, xylanase, xylanase accessory enzymes (for example, arabinofuranosidase, ferulic acid esterase, and xylan acetyl esterase), beta-glucosidase and protease. In some embodiments, the composition comprises at least about 0.0001 weight percent of the ALDC enzyme, and preferably from about 0.001 to about 5.0 weight percent of the ALDC enzyme. In some embodiments, the composition comprises at least about 0.01 to about 3.0 weight percent of the ALDC enzyme. In some embodiments, the composition comprises at least about 0.5 to about 2.0 weight percent of the ALDC enzyme. In some embodiments, the composition comprises at least about 0.8 to about 1.0 weight percent of the ALDC enzyme.

In some embodiments the compositions and methods according to the invention comprise an enzyme exhibiting ALDC activity, wherein the activity of said ALDC enzyme is in the range of about 950 to 2500 Units per mg of protein. In some embodiments the compositions and methods according to the invention comprise an enzyme exhibiting ALDC activity, wherein the activity of said ALDC enzyme is in the range of about 1000 to 2500 or about 1500 to 2500 Units per mg of protein. In some embodiments, the enzyme exhibiting ALDC activity comprises an amino acid sequence having at least 80% identity with any one selected from SEQ ID NO: 2, SEQ ID NO: 3, SEQ ID NO: 5, SEQ ID NO: 7, and SEQ ID NO: 8 or any functional fragment thereof.

In some embodiments, the ALDC enzyme is treated with, or has been treated with, glutaraldehyde to form an ALDC derivative. In some embodiments, the ALDC enzyme is treated with, or has been treated with, glutaraldehyde at a concentration corresponding to about 0.1 to about 5 g of glutaraldehyde per g of pure ALDC enzyme.

In some embodiments, the ALDC enzyme compositions described herein are used during fermentation and/or maturation of a beverage preparation process, e.g., beer, wine, cider, or perry, or sake to reduce diacetyl levels.

As used herein, the terms " beverage" and " beverage(s) product" include such foam forming fermented beverages as beer brewed with 100% malt, beer brewed under different types of regulations, ale, dry beer, near beer, light beer, low alcohol beer, low calorie beer, porter, bock beer, stout, malt liquor, non-alcoholic beer, non-alcoholic malt liquor and the like. The term "beverages" or "beverages product" also includes non-foaming beer and alternative malt beverages such as fruit flavored malt beverages, e. g., citrus flavored, such as lemon-, orange-, lime-, or berry-flavored malt beverages, liquor flavored malt beverages, e. g. , vodka-, rum-, or tequila-flavored malt liquor, or coffee flavored malt beverages, such as caffeine-flavored malt liquor, and the like. The term "beverages" or "beverages product" also includes beer made with alternative materials other than malted barley, such as rye, corn, oats, rice, millet, triticale, cassava, wheat, barley, sorghum and a combination thereof. The term "beverages" or "beverages product" also includes other fermented products such as wine or ciders or perry or sake.

Beer is traditionally referred to as an alcoholic beverage derived from malt, such as malt derived from barley grain, and optionally adjunct, such as starch containing plant material (e.g. cereal grains) and optionally flavored, e.g. with hops. In the context of the present invention, the term "beer" includes any fermented wort, produced by fermentation/brewing of a starch-containing plant material, thus in particular also beer produced exclusively from adjunct, or any combination of malt and adjunct. Beer can be made from a variety of starch-containing plant material by essentially the same process, where the starch consists mainly of glucose homopolymers in which the glucose residues are linked by alpha-1, 4- or alpha-1,6-bonds, with the former predominating. Beer can be made from alternative materials such as rye, corn, oats, rice, millet, triticale, cassava, wheat, barley, sorghum and a combination thereof

**Methods**

In some aspects the invention provides methods to improve ALDC recovery from microorganisms. In other aspects the invention provides methods for the production of ALDC in the host cells described herein

In some embodiments, the invention provides methods comprising: providing a host cell comprising a genetic alteration that causes cells of the variant strain to produce a decreased amount of a functional protease when compared to the parental cell, wherein the protease is capable of cleaving a C-Terminus and/or N-Terminus of an ALDC enzyme, and a nucleic acid encoding a heterologous ALDC enzyme in operable combination with a promoter; and cultivating the transformed host cell under conditions suitable for the production of the heterologous ALDC enzyme. In some embodiments, the protease is capable of cleaving at corresponding C-terminus position 275 of SEQ ID No:5 or SEQ ID No:2 or SEQ ID No:7. In some embodiments, the protease is capable of cleaving at corresponding C-terminus position 276 of SEQ ID No:5 or SEQ ID No:2 or SEQ ID No:7. In some embodiments, the protease is capable of cleaving at corresponding N-terminus position 37 of SEQ ID No:5 or SEQ ID No:2 or SEQ ID No:7. In some embodiments, the protease is capable of cleaving at corresponding N-terminus position 38 of SEQ ID No:5 or SEQ ID No:2 or SEQ ID No:7. In some embodiments, the protease is capable of cleaving at corresponding N-terminus position 39 of SEQ ID No:5 or SEQ ID No:2 or SEQ ID No:7. In some embodiments, the protease is capable of cleaving at corresponding N-terminus position 40 of SEQ ID No:5 or SEQ ID No:2 or SEQ ID No:7. In some embodiments, the protease is capable of cleaving at corresponding N-terminus position 42 of SEQ ID No:5 or SEQ ID No:2 or SEQ ID No:7. In some embodiments, the protease is capable of cleaving at corresponding N-terminus position 43 of SEQ ID No:5 or SEQ ID No:2 or SEQ ID No:7. In some embodiments, the protease is capable of cleaving at corresponding N-terminus position 39 of SEQ ID No:5 or SEQ ID No:2 or SEQ ID No:7. In some embodiments, the protease is a neutral metalloprotease. In some embodiments, the protease is thermolysin.

In some embodiments, the methods further comprise the step of harvesting the produced heterologous ALDC enzyme.

In some embodiments, the host cell is a bacterial host such as *Bacillus.* In some embodiments the ALDC enzyme is produced by cultivation of a *Bacillus subtilis.*

In some embodiments, the invention provides methods comprising: providing a host cell comprising a genetic alteration that causes cells of the variant strain to produce a decreased amount of a functional endogenous cell wall associated protease enzyme (WprA) when compared to the parental cell, wherein the host cell is transformed with a nucleic acid encoding a heterologous ALDC enzyme in operable combination with a promoter; and cultivating the transformed host cell under conditions suitable for the production of the heterologous ALDC enzyme. In some embodiments, the invention provides methods comprising: providing a host cell comprising a genetic alteration that causes cells of the variant strain to produce a decreased amount of a functional endogenous cell wall associated protease enzyme (WprA) when compared to a parental cell, wherein the host cell is transformed with a nucleic acid that causes cells of the variant strain to overexpress an endogenous nucleic acid sequence encoding an ALDC enzyme when compared to the parental cell; and cultivating the transformed host cell under conditions suitable for the production of the ALDC enzyme. In some embodiments, the host cell is *Bacillus.* In some embodiments, the *Bacillus* is *B. subtilis.*

In some embodiments, the invention provides methods comprising: providing a host cell comprising a genetic alteration that causes cells of the variant strain to produce a decreased amount of a functional extracellular serine protease enzyme (Vpr) when compared to the parental cell, wherein the host cell is transformed with a nucleic acid encoding a heterologous ALDC enzyme in operable combination with a promoter; and cultivating the transformed host cell under conditions suitable for the production of the heterologous ALDC enzyme. In some embodiments, the invention provides methods comprising: providing a host cell comprising a genetic alteration that causes cells of the variant strain to produce a decreased amount of a functional extracellular serine protease enzyme (Vpr) when compared to a parental cell, wherein the host cell is transformed with a nucleic acid that causes cells of the variant strain to overexpress an endogenous nucleic acid sequence encoding an ALDC enzyme when compared to the parental cell; and cultivating the transformed host cell under conditions suitable for the production of the ALDC enzyme. In some embodiments, the host cell is *Bacillus.* In some embodiments, the *Bacillus* is *B. subtilis.*

In some embodiments, the invention provides methods comprising: providing a host cell comprising a genetic alteration that causes cells of the variant strain to produce a decreased amount of both a functional extracellular serine protease enzyme (Vpr) and of a functional endogenous cell wall associated protease enzyme (WprA) when compared to the parental cell, wherein the host cell is transformed with a nucleic acid encoding a heterologous ALDC enzyme in operable combination with a promoter; and cultivating the transformed host cell under conditions suitable for the production of the heterologous ALDC enzyme. In some embodiments, the invention provides methods comprising: providing a host cell comprising a genetic alteration that causes cells of the variant strain to produce a decreased amount of both a functional extracellular serine protease enzyme (Vpr) and of a functional endogenous cell wall associated protease enzyme (WprA) when compared to a parental cell, wherein the host cell is transformed with a nucleic acid that causes cells of the variant strain to overexpress an endogenous nucleic acid sequence encoding an ALDC enzyme when compared to the parental cell; and cultivating the transformed host cell under conditions suitable for the production of the ALDC enzyme. In some embodiments, the host cell is *Bacillus.* In some embodiments, the *Bacillus* is *B. subtilis.*

In some embodiments, the invention provides methods comprising: providing a host cell further comprising a genetic alteration that causes cells of the variant strain to produce a decreased amount of both an endogenous serine alkaline protease enzyme (AprE), and an endogenous extracellular neutral metalloprotease enzyme (NprE), wherein the host cell is transformed with a nucleic acid encoding a heterologous ALDC enzyme in operable combination with a promoter; and cultivating the transformed host cell under conditions suitable for the production of the heterologous ALDC enzyme. In some embodiments, the invention provides methods comprising: providing a host cell further comprising a genetic alteration that causes cells of the variant strain to produce a decreased amount of both an endogenous serine alkaline protease enzyme (AprE), and an endogenous extracellular neutral metalloprotease enzyme (NprE), wherein the host cell is transformed with a nucleic acid that causes cells of the variant strain to overexpress an endogenous nucleic acid sequence encoding an ALDC enzyme when compared to the parental cell; and cultivating the transformed host cell under conditions suitable for the production of the ALDC enzyme. In some embodiments, the host cell is *Bacillus.* In some embodiments, the *Bacillus* is *B. subtilis.*

In some embodiments, the host cell further has decreased amount of an endogenous minor extracellular serine protease enzyme (Epr). In additional embodiments, the host cell further lacks one or both of an endogenous major intracellular serine protease enzyme (IspA), and an endogenous bacillopeptidase F enzyme (Bpr). In some embodiments, the host cell is *Bacillus.* In some embodiments, the *Bacillus* is *B. subtilis.*

In some embodiments, the invention provides methods comprising: providing a host cell having decreased amounts of an endogenous serine alkaline protease enzyme (AprE), an endogenous extracellular neutral metalloprotease enzyme (NprE), an endogenous minor extracellular serine protease enzyme (Vpr), an endogenous minor extracellular serine protease enzyme (Epr), an endogenous major intracellular serine protease enzyme (IspA), and an endogenous bacillopeptidase F enzyme (Bpr), wherein the host cell is transformed with a nucleic acid encoding a heterologous ALDC enzyme in operable combination with a promoter; and cultivating the transformed host cell under conditions suitable for the production of the heterologous ALDC enzyme. In some embodiments, the invention provides methods comprising: providing a host cell having decreased amounts of an endogenous serine alkaline protease enzyme (AprE), an endogenous extracellular neutral metalloprotease enzyme (NprE), an endogenous minor extracellular serine protease enzyme (Vpr), an endogenous minor extracellular serine protease enzyme (Epr), an endogenous major intracellular serine protease enzyme (IspA), and an endogenous bacillopeptidase F enzyme (Bpr), wherein the host cell is transformed with a nucleic acid that causes cells of the variant strain to overexpress an endogenous nucleic acid sequence encoding an ALDC enzyme when compared to the parental cell; and cultivating the transformed host cell under conditions suitable for the production of the ALDC enzyme. In some embodiments, the host cell is *Bacillus.* In some embodiments, the *Bacillus* is *B. subtilis.*

In some embodiments, the invention provides methods comprising: providing a host cell having decreased amounts of an endogenous serine alkaline protease enzyme (AprE), an endogenous extracellular neutral metalloprotease enzyme (NprE), an endogenous cell wall associated protease enzyme (WprA), an endogenous minor extracellular serine protease enzyme (Epr), an endogenous major intracellular serine protease enzyme (IspA), and an endogenous bacillopeptidase F enzyme (Bpr), wherein the host cell is transformed with a nucleic acid encoding a heterologous ALDC enzyme in operable combination with a promoter; and cultivating the transformed host cell under conditions suitable for the production of the heterologous ALDC enzyme. In some embodiments, the invention provides methods comprising: providing a host cell having decreased amounts of an endogenous serine alkaline protease enzyme (AprE), an endogenous extracellular neutral metalloprotease enzyme (NprE), an endogenous cell wall associated protease enzyme (WprA), an endogenous minor extracellular serine protease enzyme (Epr), an endogenous major intracellular serine protease enzyme (IspA), and an endogenous bacillopeptidase F enzyme (Bpr), wherein the host cell is transformed with a nucleic acid that causes cells of the variant strain to overexpress an endogenous nucleic acid sequence encoding an ALDC enzyme when compared to the parental cell; and cultivating the transformed host cell under conditions suitable for the production of the ALDC enzyme. In some embodiments, the host cell is *Bacillus.* In some embodiments, the *Bacillus* is *B. subtilis.*

In some embodiments, the invention provides methods comprising: providing a host cell having decreased amounts of an endogenous serine alkaline protease enzyme (AprE), an endogenous extracellular neutral metalloprotease enzyme (NprE), an endogenous minor extracellular serine protease enzyme (Vpr), an endogenous minor extracellular serine protease enzyme (Epr), an endogenous major intracellular serine protease enzyme (IspA), an endogenous bacillopeptidase F enzyme (Bpr), and an endogenous cell wall associated protease enzyme (WprA), wherein the host cell is transformed with a nucleic acid encoding a heterologous ALDC enzyme in operable combination with a promoter; and cultivating the transformed host cell under conditions suitable for the production of the heterologous ALDC enzyme. In some embodiments, the invention provides methods comprising: providing a host cell having decreased amounts of an endogenous serine alkaline protease enzyme (AprE), an endogenous extracellular neutral metalloprotease enzyme (NprE), an endogenous minor extracellular serine protease enzyme (Vpr), an endogenous minor extracellular serine protease enzyme (Epr), an endogenous major intracellular serine protease enzyme (IspA), an endogenous bacillopeptidase F enzyme (Bpr), and an endogenous cell wall associated protease enzyme (WprA), wherein the host cell is transformed with a nucleic acid that causes cells of the variant strain to overexpress an endogenous nucleic acid sequence encoding an ALDC enzyme when compared to the parental cell; and cultivating the transformed host cell under conditions suitable for the production of the ALDC enzyme. In some embodiments, the host cell is *Bacillus.* In some embodiments, the *Bacillus* is *B. subtilis.*

In additional embodiments, the host cell further has decreased amounts of one or more additional proteases, such ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, ywaD, or other proteases known to those of skill in the art. In some embodiments, the host cell is *Bacillus.* In some embodiments, the *Bacillus* is *B. subtilis.*

In some embodiments, a method of producing acetoin is provided in the disclosure. In some embodiments, a method of decomposing acetolactate is provided in the disclosure. The methods involve the step of treating a substrate with an ALDC enzyme composition as described herein.

In some embodiments, ALDC enzyme comprises an amino acid sequence having at least 80% identity with any one selected from SEQ ID NO: 2, SEQ ID NO: 3, SEQ ID NO: 5, SEQ ID NO: 7, and SEQ ID NO: 8 or any functional fragment thereof.

In some embodiments a method of producing acetoin during the production of a fermented beverage is provided in the disclosure. In some embodiments, a method of decomposing acetolactate during the production of a fermented beverage is provided in the disclosure.

*Fermented Products*

In one aspect the present invention relates to a process for producing fermented alcoholic products with a low diacetyl content by fermentation of a carbohydrate containing substrate with a microorganism. As used herein, a fermented alcoholic product with "low diacetyl content" refers to a fermented alcoholic product (e.g. a beer and/or a wine and/or a cider and/or a perry and/or sake) produced by fermentation of a carbohydrate containing substrate with a host cell as described herein and/or the ALDC and/or the ALDC derivative compositions described herein wherein the diacetyl levels are lower when compared to the fermented alcoholic produced by fermentation of a carbohydrate containing substrate in the absence of a host cell described herein and/or the ALDC and/or the ALDC derivative compositions described herein under the same fermentation conditions (e.g. same temperature and for the same length of time). Examples of fermented alcoholic products with low diacetyl content are fermented alcoholic products in which the diacetyl levels are less than about 1 ppm and/or the diacetyl levels are below about 0.5 mg/L. In one embodiment, the diacetyl levels are less than about 0.5 ppm, or less than about 0.1 ppm, or less than about 0.05 ppm, or less than about 0.01 ppm, or less than about 0.001 ppm. In one embodiment, the diacetyl levels are about less than 0.1 mg/L, or about less than 0.05 mg/L, or about less than 0.01 mg/L or about less than 0.001 mg/L.

When carbohydrate containing substrates, such as wort (e.g. worts with low malt) content or fruit juices (such as grape juice, apple juice or pear juice), are fermented with yeast or other microorganisms, various processes take place in addition to the alcohol fermentation which may cause generation of undesired by-products, e.g., the formation of diacetyl which has a strong and unpleasant smell even in very low concentrations. Alcoholic beverages, such as beer or wine or cider or perry or sake, may thus have an unacceptable aroma and flavor if the content of diacetyl considerably exceeds certain limits, e.g., in the case of some beers about 0.1 ppm.

Formation of diacetyl is also disadvantageous in the industrial production of ethanol because it is difficult to separate diacetyl from ethanol by distillation. A particular problem arises in the preparation of absolute ethanol where ethanol is dehydrated by azeotropic distillation with benzene. Diacetyl will accumulate in the benzene phase during the azeotropic distillation which may give rise to mixtures of diacetyl and benzene which makes it difficult to recover the benzene used for the azeotropic distillation.

The conventional brewing of beer comprises fermenting the wort with a suitable species of yeast, such as *Saccharomyces cerevisae* or *Saccharomyces carlsbergensis.*

Typically in conventional brewing, the fermentation is usually effected in two steps, a main fermentation of a duration of normally 5 to 12 days and a secondary fermentation-a so-called maturation process-which may take from up to 12 weeks. During the main fermentation most of the carbohydrates in the wort are converted to ethanol and carbon dioxide. Maturation is usually effected at a low temperature in the presence of a small residual amount of yeast. The purposes of the maturation are, inter alia, to precipitate undesirable, high molecular weight compounds and to convert undesirable compounds to compounds, such as diols, which do not affect flavor and aroma. For example butanediol, the final product of the conversion of a-acetolactate and diacetyl in beer, is typically reported as a compound with neutral sensory characteristics.

In some aspects, the present invention relates to the use of a host cell described herein and/or a composition as described herein in beer and/or wine and/or cider and/or perry and/or sake fermentation. In some embodiments, the present invention comprises the use of the host cells described herein and/or the ALDC compositions described herein to decompose acetolactate during beer and/or wine and/or cider and/or perry and/or sake fermentation or maturation. Also, the invention comprises the use of the host cells described herein and/or ALDC derivative according to the description to decompose acetolactate during beer and/or wine and/or cider and/or perry and/or sake fermentation or maturation.

In some embodiments, the methods of the invention are thus characterized by the treatment of a substrate with a host cell as described herein and/or a composition comprising ALDC or an ALDC derivative as described herein during or in continuation of a fermentation process, e.g., maturation.

Thus, in some embodiments, acetolactate is enzymatically decarboxylated to acetoin, the result being that when undesirable, the formation of diacetyl from acetolactate is avoided. In some embodiments, other enzymes are used in combination with the host cell and/or the ALDC for the conversion of $\alpha$-acetolactate. Examples of such enzymes include, but are not limited to, acetolactate reductoisomerases or isomerases.

In some embodiments, the host cells described herein and/or the ALDC and/or ALDC derivative compositions described herein are used together with ordinary yeast in a batch fermentation.

Instead of using the enzyme in a free state, it may be used in an immobilized state, the immobilized enzyme being added to the wort during or in continuation of the fermentation (e.g., during maturation). The immobilized enzyme may also be maintained in a column through which the fermenting wort or the beer is passed. The enzyme may be immobilized separately, or coimmobilized yeast cells and acetolactate decarboxylase may be used.

In some embodiments, the host cells as described herein and/or the ALDC and/or ALDC derivative compositions described herein are used during beer and/or wine and/or cider and/or perry and/or sake fermentation or maturation to reduce the diacetyl levels to below about 1 ppm, or about less than 0.5 ppm, or about less than 0.1 ppm, or less than 0.05 ppm or less than 0.01 ppm, or about less than 0.001 ppm. In some embodiments, the host cells as described herein and/or the ALDC and/or ALDC derivative compositions described herein are used during beer and/or wine and/or cider and/or perry and/or sake fermentation or maturation to reduce the diacetyl levels to below 0.1 ppm.

In some embodiments, the host cells as described herein and/or the ALDC and/or ALDC derivative compositions described herein are used during beer and/or wine and/or cider and/or perry and/or sake fermentation or maturation to reduce VDK content below 0.1 mg/L, or about less than 0.05 mg/L, or less than 0.01 mg/L or less than 0.001 mg/L. Total VDK refers to the amount of Diacetyl plus 2,3-pentanedione. In some embodiments, the host cells as described herein and/or the ALDC and/or ALDC derivative compositions described herein are used during beer and/or wine and/or cider and/or perry and/or sake fermentation or maturation to reduce Total VDK content below 0.1 mg/L.

In some embodiments, the host cells as described herein and/or the ALDC and/or ALDC derivative compositions described herein are used during beer and/or wine and/or cider and/or perry and/or sake fermentation or maturation to reduce the diacetyl levels to below about 0.5 mg/L, or about less than 0.1 mg/L, or about less than 0.05 mg/L, or less than 0.01 mg/L or less than 0.001 mg/L. In some embodiments, the host cells as described herein and/or the ALDC and/or ALDC derivative compositions described herein are used during beer and/or wine and/or cider and/or perry and/or sake fermentation or maturation to reduce the diacetyl levels to below 0.1 mg/L.

In some embodiments, the host cells as described herein and/or the ALDC and/or ALDC derivative compositions described herein are used during beer and/or wine and/or cider and/or perry and/or sake fermentation or maturation to reduce other vicinal diketones to below 0.1 mg/L, or about less than 0.05 mg/L, or less than 0.01 mg/L or less than 0.001 mg/L.

The processes of the invention can not only be used in connection with the brewing of beer, but is also suitable for the production of any suitable alcoholic beverage where a reduction in diacetyl levels or other vicinal diketones is desirable (e.g. wine, sake, cider, perry, etc...). In some embodiments, the processes of the invention can be used in the production of wine where similar advantages are obtained, in particular a reduction in the maturation period and a simplification of the process. Of special interest in this context is the use of acetolactate converting enzymes in connection with the so-called malo-lactic fermentation. This process which is effected by microorganisms as species of Leuconostoc, Lactobacillus or Pediococcus is carried out after the main fermentation of wine in order to increase the pH of the product as well as its biological stability and to develop the flavor of the wine. Moreover, it is highly desirable to carry out the fermentation since it makes possible rapid bottling and thereby improves the cash-flow of wineries substantially. Unfortunately, however, the process may give rise to off-flavors due to diacetyl, the formation of which can be reduced with the aid of acetolactate converting enzymes.

Thus, in some embodiments, the processes of the invention provide for the production of alcoholic beverages with lower content of diacetyl when compared to a process without the use of the host cells as described herein and/or the ALDC and/or ALDC derivative compositions described herein, wherein the time required for producing the alcoholic beverages with lower content of diacetyl is reduced by at least 10%, or at least 20% or at least 30%, or at least 40%, or at least 50%, or at least 60%, or at least 70%, or at least 80%, or at least 90% when compared to a process without the use of the host cells as described herein and/or the ALDC and/or ALDC derivative compositions described herein. In some embodiments, the processes of the invention provide for the production of alcoholic beverages with lower content of diacetyl when compared to a process without the use of the host cells as described herein and/or the ALDC and/or ALDC derivative compositions described herein, wherein a maturation step is completely eliminated.

In some embodiments, the host cells as described herein and/or the ALDC and/or ALDC derivative compositions described herein are used during a fermentation process (e.g. beer and/or wine and/or cider and/or perry and/or sake fermentation), such that the time required for the fermentation process is reduced by at least 10%, or at least 20% or at least 30%, or at least 40%, or at least 50%, or at least 60%, or at least 70%, or at least 80%, or at least 90%, when compared to a process without the host cells as described herein and/or the use of the ALDC and/or ALDC derivative compositions described herein. In some embodiments, the processes of the invention provide for the production of alcoholic beverages with lower content of diacetyl when compared to a process without the use of the host cells as described herein and/or the ALDC and/or ALDC derivative compositions described herein, wherein a maturation step is completely eliminated.

In some embodiments, the host cells as described herein and/or the ALDC and/or ALDC derivative compositions described herein are used during a maturation or conditioning process (e.g. beer maturation/conditioning), such that the time required for the maturation or conditioning process is reduced by at least 10%, or at least 20% or at least 30%, or at least 40%, or at least 50%, or at least 60%, or at least 70%, or at least 80%, or at least 90%, when compared to a process without the use of the host cells as described herein and/or the ALDC and/or ALDC derivative compositions described herein. In some embodiments, the processes of the invention provide for the production of alcoholic beverages with lower content of diacetyl when compared to a process without the use of the host cells as described herein and/or the ALDC and/or ALDC derivative compositions described herein, wherein a maturation step is completely eliminated.

Further, in some embodiments, the processes described herein can be used to advantage for industrial preparation of ethanol as fermentation products are obtained without or practically without any content of diacetyl, which simplifies the distillation process, especially in case of azeotropic for the preparation of absolute ethanol, i.e. pure anhydrous ethanol.

In some embodiments, the invention provides methods for beer and/or wine and/or cider and/or perry and/or sake production comprising adding one or more host cells as described herein and/or an ALDC enzyme/or ALDC derivative compositions described herein

**Production of ALDC enzymes**

In one aspect, the invention relates to a host cell having heterologous expression of an ALDC enzyme as herein described. In another aspect, the host cell comprises, preferably transformed with, a plasmid or an expression vector as described herein. In another aspect, the invention relates to a host cell transformed with a nucleic acid that causes the host cell to overexpress an endogenous nucleic acid sequence encoding an ALDC enzyme when compared to the parental cell.

In some embodiments, the host cell is a bacterial host such as *Bacillus.* In some embodiments the ALDC enzyme is produced by cultivation of a *Bacillus subtilis* strain containing a gene encoding and expressing an ALDC enzyme (e.g. ALDC of *Bacillus brevis).* Examples of such host cells and cultivation thereof are described in DK149335B. In some embodiments the ALDC enzyme is produced by cultivation of a *Bacillus subtilis* strain containing a gene encoding and overexpressing an ALDC enzyme.

Examples of suitable expression and/or integration vectors are provided in Sambrook et al. (1989) *supra,* and Ausubel (1987) *supra,* and van den Hondel et al. (1991) in Bennett and Lasure (Eds.) More Gene Manipulations In Fungi, Academic Press pp. 396-428 and U.S. Patent No. 5,874,276. Reference is also made to the Fungal Genetics Stock Center Catalogue of Strains (FGSC, http://www.fgsc.net) for a list of vectors. Particularly useful vectors include vectors obtained from for example Invitrogen and Promega. Suitable plasmids for use in bacterial cells include pBR322 and pUC19 permitting replication in *E. coli* and pE194 for example permitting replication in *Bacillus.* Other specific vectors suitable for use in *E. coli* host cells include vectors such as pFB6, pBR322, pUC18, pUC100, pDONR™201, 10 pDONR™221, pENTR™, pGEM®3Z and pGEM®4Z.

In some embodiments, the host cells will be gram-positive bacterial cells. Non-limiting examples include strains of *Streptomyces* (*e.g., S. lividans, S. coelicolor,* and *S. griseus*) and *Bacillus.* As used herein, "the genus *Bacillus"* includes all species within the genus *"Bacillus,"* as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus tearothermophilus*."

In some embodiments, the host cell is a gram-negative bacterial strain, such as *E. coli* or *Pseudomonas sp.* In other embodiments, the host cells may be yeast cells such as *Saccharomyces sp., Schizosaccharomyces sp., Pichia sp.,* or *Candida sp.* In other embodiments, the host cell will be a genetically engineered host cell wherein native genes have been inactivated, for example by deletion in bacterial or fungal cells. Where it is desired to obtain a fungal host cell having one or more inactivated genes known methods may be used (*e.g.,* methods disclosed in U.S. Patent No. 5,246,853, U.S. Patent No. 5,475,101, and WO 92/06209). Gene inactivation may be accomplished by complete or partial deletion, by insertional inactivation or by any other means that renders a gene nonfunctional for its intended purpose (such that the gene is prevented from expression of a functional protein). In other embodiments, the host cell is a protease deficient or protease minus strain.

Introduction of a DNA construct or vector into a host cell includes techniques such as transformation; electroporation; nuclear microinjection; transduction; transfection, (e.g., lipofection-mediated and DEAE-Dextrin mediated transfection); incubation with calcium phosphate DNA precipitate; high velocity bombardment with DNA-coated microprojectiles; and protoplast fusion. General transformation techniques are known in the art (*see, e.g.,* Ausubel et al. (1987) *supra,* chapter 9; and Sambrook et al. (1989) *supra,* and Campbell et al., Curr. Genet. 16:53-56 (1989)).

Transformation methods for *Bacillus* are disclosed in numerous references including Anagnostopoulos C. and J. Spizizen, J. Bacteriol. 81:741-746 (1961) and WO 02/14490.

In one aspect, the invention relates to a method of isolating an ALDC enzyme as defined herein, the method comprising the steps of inducing synthesis of the ALDC enzyme in a host cell as defined herein having heterologous expression of said ALDC enzyme and recovering extracellular protein secreted by said host cell, and optionally purifying the ALDC enzyme. In one aspect, the invention relates to a method of isolating an ALDC enzyme as defined herein, the method comprising the steps of inducing synthesis of the ALDC enzyme in a host cell as defined herein having homologous expression of said ALDC enzyme and recovering extracellular protein secreted by said host cell, and optionally purifying the ALDC enzyme. In a further aspect, the invention relates to a method for producing an ALDC enzyme as defined herein, the method comprising the steps of inducing synthesis of the ALDC enzyme in a host cell as defined herein having heterologous expression of said ALDC enzyme, and optionally purifying the ALDC enzyme. In a further aspect, the invention relates to a method for producing an ALDC enzyme as defined herein, the method comprising the steps of inducing synthesis of the ALDC enzyme in a host cell as defined herein having homologous expression of said ALDC enzyme, and optionally purifying the ALDC enzyme. In a further aspect, the invention relates to a method of expressing an ALDC enzyme as defined herein, the method comprising obtaining a host cell as defined herein, or any suitable host cell as known by a person of ordinary skill in the art, and expressing the ALDC enzyme from said host cell, and optionally purifying the ALDC enzyme. In another aspect, the ALDC enzyme as defined herein is the dominant secreted protein.

In some embodiments, metal ions such as $Zn^{2+}$, $Mg^{2+}$, $Mn^{2+}$, $Co^{2+}$, $Cu^{2+}$, $Ba^{2+}$, $Ca^{2+}$ and $Fe^{2+}$ and combinations thereof are added to the cultivation media during and/or after ALDC production to increase the recovered yields from microorganisms and/or to increase the stability of the ALDC and/or to increase the activity of the ALDC. In some embodiments, the metal ion is added to the cultivation media so that said metal ion is present in said media at a concentration of about 1 $\mu$M to about 1 mM, such as about 25 $\mu$M to about 150 $\mu$M, or about 40 $\mu$M to about 150 $\mu$M, or about 60 $\mu$M to about 150 $\mu$M, or about 25 $\mu$M to about 100 $\mu$M, or about 25 $\mu$M to about 50 $\mu$M, or 30 $\mu$M to about 40 $\mu$M. In some embodiments, metal ions such as $Zn^{2+}$, $Mg^{2+}$, $Mn^{2+}$, $Co^{2+}$, $Cu^{2+}$, $Ba^{2+}$, $Ca^{2+}$ and $Fe^{2+}$ and combinations thereof are added to the media (such as a fermentation and/or a maturation media) during and/or after ALDC production to increase the recovered yields from microorganisms and/or to increase the stability of the ALDC and/or to increase the activity of the ALDC. In some embodiments, the metal ion is added to the fermentation and/or maturation media so that said metal ion is present in said media at a concentration of about 1 $\mu$M to about 300 $\mu$M, such as about 6 $\mu$M to about 300 $\mu$M, about 1 $\mu$M to about 100 $\mu$M, about 1 $\mu$M to about 50 $\mu$M, about 1 $\mu$M to about 25 $\mu$M, or about 6 $\mu$M to about 25 $\mu$M. The term "cultivation media" as used herein refers to a media which supports the growth of microorganisms such as an ALDC producing host cell. Examples of a cultivation media include: media based on MOPs buffer with, for instance, urea as the major nitrogen source and maltrin as the main carbon source; and TSB broth. The term "fermentation media" as used herein refers to a medium comprising carbohydrate containing substrates which can be fermented by yeast or other microorganisms to produce, for example, beer or wine or cider or perry or sake. Examples of fermentation media include: wort and fruit juices (such as grape juice, apple juice and pear juice). The term "maturation media" as used herein refers to a medium comprising carbohydrate containing substrates which have been fermented by yeast or other microorganisms to produce, for example, beer or wine or cider or perry or sake. Examples of maturation media include partially fermented wort and fruit juices (such as grape juice, apple juice and pear juice). The term "increase the stability" as used in connection to media to which metal ions are added refers to an ALDC enzyme whose ALDC activity is maintained for a longer period of time when in the presence of a metal ion (such as $Zn^{2+}$) when compared to the ALDC activity of the enzyme in the absence of the metal ion in the media. The term "increase the activity" as used in connection to media to which metal ions are added refers to an ALDC enzyme having an increased ALDC activity when in the presence of a metal ion (such as $Zn^{2+}$) when compared to the ALDC activity of the enzyme in the absence of the metal ion in the media. The term "improved" in connection to the yield of ALDC enzyme in media to which metal ions are added refers to an increase in the ALDC activity which is produced when a host microorganism is in the presence of a metal ion (such as $Zn^{2+}$) compared to the ALDC activity produced when the host microorganism is in the absence of the metal ion. Without wishing to be bound by theory, the metal ion (such as $Zn^{2+}$) can be added during and/or after the culture process (e.g. ALDC production) in order to increase stability and/or increase activity and/or to increase yield of ALDC enzymes.

In some embodiments, the invention provides a cultivation media for an ALDC producing host cell comprising a metal ion at a concentration of about 1 $\mu$M to about 1 mM (such as about 25 $\mu$M to about 150 $\mu$M, or about 40 $\mu$M to about 150 $\mu$M, or about 60 $\mu$M to about 150 $\mu$M, or about 25 $\mu$M to about 100 $\mu$M, or about 25 $\mu$M to about 50 $\mu$M, or 30 $\mu$M to about 40 $\mu$M). In some embodiments, the invention provides a cultivation media for an ALDC producing host cell comprising a metal ion at a concentration of about 25 $\mu$M to about 100 $\mu$M. In some embodiments, the invention provides a cultivation media for an ALDC producing host cell comprising a metal ion at a concentration of about 25 $\mu$M to about 50 $\mu$M. In some embodiments, the metal ion is selected from the group consisting of $Zn^{2+}$, $Mg^{2+}$, $Mn^{2+}$, $Co^{2+}$, $Cu^{2+}$, $Ba^{2+}$, $Ca^{2+}$ and $Fe^{2+}$ and combinations thereof. In some embodiments, the metal ion is selected from the group consisting $Zn^{2+}$, $Mn^{2+}$, and $Co^{2+}$. In some embodiments, the metal ion is selected from the group consisting $Zn^{2+}$, $Cu^{2+}$, and $Fe^{2+}$. In some embodiments, the metal ion is $Zn^{2+}$. Zinc sulfate ($ZnSO_4$) is example of a source of $Zn^{2+}$ ions. Magnesium sulfate ($MgSO_4$) is an example of a source of $Mg^{2+}$ ions. Manganese(II) sulfate ($MnSO_4$) is an example of a source of $Mn^{2+}$ ions. Cobalt(II)chloride ($CoCl_2$) is an example of a source of $Co^{2+}$ ions. Copper(II) sulphate ($CuSO_4$) is an example of a source of $Cu^{2+}$ ions. Barium sulfate ($BaSO_4$) is an example of a source of $Ba^{2+}$ ions. Calcium sulfate ($CaSO_4$) is an example of a source of $Ca^{2+}$ ions. Iron(II) sulfate ($FeSO_4$) is an example of a source of $Fe^{2+}$ ions. In some embodiments, the activity of said ALDC enzyme is in the range of 950 to 2500 Units per mg of protein. In some embodiments, the activity of said ALDC enzyme is in the range of 1000 to 2500 or 1500 to 2500 Units per mg of protein. The term "ALDC producing host cell" as used herein refers to a host cell capable of expressing ALDC enzyme when said host cell is cultured under conditions permitting the expression of the nucleic acid sequence encoding ALDC. The nucleic acid sequence encoding ALDC enzyme may be heterologous or homologous to the host cell. The "ALDC producing host strain" as referred to herein is a protease deficient or protease minus strain. In some embodiments, the ALDC producing host cell is *Bacillus subtilis*. In some embodiments, the ALDC producing host cell is *Bacillus subtilis* comprising a gene encoding and expressing ALDC enzyme wherein the ALDC enzyme comprises an amino acid sequence having at least 70%, 75%, 80%, 85%, 90%, 95%, 97%, 98%, 99% or 100% identity with SEQ ID NO: 2, SEQ ID NO: 3, SEQ ID NO: 5, SEQ ID NO: 7, SEQ ID NO: 8, or any functional fragment thereof. In some embodiments, the ALDC producing host cell is *Bacillus subtilis* comprising a nucleic acid sequence encoding ALDC wherein said nucleic acid sequence encoding ALDC has at least 70%, 75%, 80%, 85%, 90%, 95%, 97%, 98%, 99% or 100% identity with SEQ ID NO: 1, SEQ ID NO: 4, SEQ ID NO: 6 or any functional fragment thereof. In some embodiments, the ALDC producing host cell is *Bacillus subtilis* comprising a gene encoding ALDC derived from *Bacillus brevis.*

**EXAMPLES**

The present disclosure is described in further detail in the following examples, which are not in any way intended to limit the scope of the disclosure as claimed. The attached figures are meant to be considered as integral parts of the specification and description of the disclosure. The following examples are offered to illustrate, but not to limit the claimed disclosure.

**Example 1 - Heterologous expression of acetolactate decarboxylase, aldB**

The *Brevibacillus brevis* (which may be referred to as *Bacillus brevis)* acetolactate decarboxylases (ALDC) *aldB* gene was previously identified (Diderichsen et al., J Bacteriol. (1990) 172(8): 4315), with the sequence set forth as UNIPROT Accession No. P23616.1. The sequence of this gene, *aldB,* is depicted in SEQ ID NO:1. The nucleotides highlighted in bold and underlined are the nucleotides which encode the signal peptide.

*SEQ ID NO: 1 sets forth the nucleotide sequence of the aldB gene:*

**<u>atgaaaaaaaatatcatcacttctatcacatctctggctctggttgccgggctgtctttgactgcttttgca</u>**gctacaacggctactgtac

cagcaccacctgccaagcaggaatccaaacctgcggttgccgctaatccggcaccaaaaaatgtactgtttcaatactcaacgatcaatgca

ctcatgcttggacagtttgaaggggacttgactttgaaagacctgaagctgcgaggcgatatggggcttggtaccatcaatgatctcgatgga

gagatgattcagatgggtacaaaattctaccagatcgacagcaccggaaaattatcggagctgccagaaagtgtgaaaactccatttgcggt

tactacacatttcgagccgaaagaaaaaactacattaaccaatgtgcaagattacaatcaattaacaaaaatgcttgaggagaaatttgaaaac

aagaacgtctttatgccgtaaagctgaccggtacctttaagatggtaaaggctagaacagttccaaaacaaaccagaccttatccgcagctg

actgaagtaaccaaaaaacaatccgagtttgaatttaaaaatgttaagggaaccctgattggcttctatacgccaaattatgcagcagccctga

atgttcccggattccatctccacttcatcacagaggataaaacaagtggcggacacgtattaaatctgcaatttgacaacgcgaatctggaaat

ttctccgatccatgagtttgatgtacaattgccgcacacagatgatttttgcccactctgatctgacacaagttactactagccaagtacaccaag

ctgagtcagaaagaaaataa

*SEQ ID NO: 6 sets forth an example of a nucleotide sequence encoding an acetolactate decarboxylase - the nucleotides highlighted in bold and underlined are the nucleotides which encode the signal peptide:*

**atgaaaaaaaatatcatcacttctatcacatctctggctctcgttgccgggctgtctttgactgctttgcagctacaacggctactgta**

**ccagcaccacctgccaagcaggaatccaaacctgtggttgccgctaatccggcaccaaaaaatgtactgtttcaatactcaacgat**

**caatgcactcatgcttggacagtttgaaggggacttgactttgaaagacctgaagctacgaggcgatatggggcttggt**accatcaa

tgatctcgatggagagatgattcagatgggtacaaaattctaccagatcgacagcaccggaaaattatccgagctgccagaaagtgtgaaaa

ctccatttgcggttactacacatttcgagccgaaagaaaaaactacattaaccaatgtgcaagattacaatcaattaacaaaaatgcttgagga

gaaatttgaaacaagaacgtcttttatgccgtaaagctgaccggtacctttaagatggtaaaggctagaacagttccaaaacaaaccagacc

ttatccgcagctgactgaagtaaccaaaaaacaatccgagtttgaatttaaaaatgttaagggaaccctgattggcttctatacgccaaattatg

cagcagccctgaatgttcccggattccatctccacttcatcacagaggataaaacaagtggcggacacgtattaaatctgcaatttgacaacg

cgaatctggaaatttctccgatccatgagtttgatgtacaattgccgcacacagatgattttgcccactctgatctgacacaagttactactagcc

aagtacaccaagctgagtcagaaagaaataa

The proenzyme encoded by the *aldB* gene is depicted in SEQ ID NO: 2. At the N-terminus, the protein has a signal peptide with a length of 24 amino acids as predicted by SignalP-NN (Emanuelsson et al., Nature Protocols (2007) 2: 953-971). This signal peptide sequence is underlined and is in bold in SEQ ID NO:2. The presence of a signal peptide indicates that this acetolactate decarboxylase is a secreted enzyme. The sequence of the predicted, fully processed mature chain (aldB, 261 amino acids) is depicted in SEQ ID NO: 3.

*SEQ ID NO: 2 sets forth the amino acid sequence of the acetolactate decarboxylase (ALDC) precursor aldB:*

**MKKNIITSITSLALVAGLSLTAFA**ATTATVPAPPAKQESKPAVAANPAPKNVLFQYSTI

NALMLGQFEGDLTLKDLKLRGDMGLGTINDLDGEMIQMGTKFYQIDSTGKLSELPESV

KTPFAVTTHFEPKEKTTLTNVQDYNQLTKMLEEKFENKNVFYAVKLTGTFKMVKARTV

PKQTRPYPQLTEVTKKQSEFEFKNVKGTLIGFYTPNYAAALNVPGFHLHFITEDKTSGGH

VLNLQFDNANLEISPIHEFDVQLPHTDDFAHSDLTQVTTSQVHQAESERK

*SEQ ID NO: 7 sets forth an example of an amino acid sequence of the acetolactate decarboxylase (ALDC) precursor aldB - the signal peptide sequence is underlined and is in bold:*

**MKKNIITSITSLALVAGLSLTAFA**ATTATVPAPPAKQESKPVVAANPAPKNVLFQYSTI

NALMLGQFEGDLTLKDLKLRGDMGLGTINDLDGEMIQMGTKFYQIDSTGKLSELPESV

KTPFAVTTHFEPKEKTTLTNVQDYNQLTKMLEEKFENKNVFYAVKLTGTFKMVKARTV

PKQTRPYPQLTEVTKKQSEFEFKNVKGTLIGFYTPNYAAALNVPGFHLHFITEDKTSGGH

VLNLQFDNANLEISPIHEFDVQLPHTDDFAHSDLTQVTTSQVHQAESERK

*SEQ ID NO: 3 sets forth the predicted amino acid sequence of the mature acetolactate decarboxylase (ALDC) aldB (261 amino acids):*

ATTATVPAPPAKQESKPAVAANPAPKNVLFQYSTINALMLGQFEGDLTLKDLKLRGDM

GLGTINDLDGEMIQMGTKFYQIDSTGKLSELPESVKTPFAVTTHFEPKEKTTLTNVQDYN

QLTKMLEEKFENKNVFYAVKLTGTFKMVKARTVPKQTRPYPQLTEVTKKQSEFEFKNV

KGTLIGFYTPNYAAALNVPGFHLHFITEDKTSGGHVLNLQFDNANLEISPIHEFDVQLPHT

DDFAHSDLTQVTTSQVHQAESERK

*SEQ ID NO: 8 sets forth an example of the predicted amino acid sequence of the mature acetolactate decarboxylase (ALDC) aldB (261 amino acids):*

ATTATVPAPPAKQESKPVVAANPAPKNVLFQYSTINALMLGQFEGDLTLKDLKLRGDM

GLGTINDLDGEMIQMGTKFYQIDSTGKLSELPESVKTPFAVTTHFEPKEKTTLTNVQDYN

QLTKMLEEKFENKNVFYAVKLTGTFKMVKARTVPKQTRPYPQLTEVTKKQSEFEFKNV

KGTLIGFYTPNYAAALNVPGFHLHFITEDKTSGGHVLNLQFDNANLEISPIHEFDVQLPHT

DDFAHSDLTQVTTSQVHQAESERK

The *aldB* gene that encodes an acetolactate decarboxylases enzyme (ALDC) was produced in *B. subtilis* using an expression cassette consisting of the *B. subtilis aprE* promoter, the *B. subtilis aprE* signal peptide sequence, the mature aldB and a BPN' terminator. This cassette was cloned into the pBN based replicating shuttle vector (Babe' et al. (1998), Biotechnol. Appl. Biochem. 27: 117-124) and transformed into a *B. subtilis* strain.

A map of the pBN vector containing the aldB gene (pBN- Bbrev-ssoU) is shown in Figure 1.

To produce aldB, a *B. subtilis* strain transformant containing pBN- aldB was cultured in 15 ml Falcon tubes for 16 hours in TSB (broth) with 10 ppm neomycin, and 300 $\mu$l of this pre-culture was added to a 500 mL flask filled with 30 mL of cultivation media (described below) supplemented with 10 ppm neomycin. The flasks were incubated for 24, 48 and 72 hours at 33°C with constant rotational mixing at 180 rpm. Cultures were harvested by centrifugation at 14500 rpm for 20 minutes in conical tubes. The culture supernatants were used for protein determination and assays. The cultivation media was an enriched semi-defined media based on MOPs buffer, with urea as major nitrogen source, glucose as the main carbon source, and supplemented with 1% soytone for robust cell growth.

The nucleotide mature sequence of the aldB gene in plasmid pBN- aldB is depicted in SEQ ID NO:4

gctacaacggctactgtaccagcaccacctgccaagcaggaatccaaacctgcggttgccgctaatccggcaccaaaaaatgtactgtttc

aatactcaacgatcaatgcactcatgcttggacagtttgaaggggacttgactttgaaagacctgaagctgcgaggcgatatggggcttggta

ccatcaatgatctcgatggagagatgattcagatgggtacaaaattctaccagatcgacagcaccggaaaattatcggagctgccagaaagt

gtgaaaactccatttgcggttactacacatttcgagccgaaagaaaaaactacattaaccaatgtgcaagattacaatcaattaacaaaaatgc

ttgaggagaaatttgaaaacaagaacgtcttttatgccgtaaagctgaccggtacttttaagatggtaaaggctagaacagttccaaaacaaa

ccagaccttatccgcagctgactgaagtaaccaaaaaacaatccgagtttgaatttaaaaatgttaagggaaccctgattggcttctatacgcc

aaattatgcagcagccctgaatgttcccggattccatctccacttcatcacagaggataaaacaagtggcggacacgtattaaatctgcaattt

gacaacgcgaatctggaaatttctccgatccatgagtttgatgttcaattgccgcacacagatgattttgcccactctgatctgacacaagttact

actagccaagtacaccaagctgagtcagaaagaaaa

The amino acid sequence of the aldB precursor protein expressed from plasmid pBN-aldB is depicted in SEQ ID NO:5

*vrskklwisllfaltliftmafsnmsaqa*ATTATVPAPPAKQESKPAVAANPAPKNVLFQYSTINALML GQFEGDLTLKDLKLRGDMGLGTINDLDGEMIQMGTKFYQIDSTGKLSELPESVKTPFAV TTHFEPKEKTTLTNVQDYNQLTKMLEEKFENKNVFYAVKLTGTFKMVKARTVPKQTRP YPQLTEVTKKQSEFEFKNVKGTLIGFYTPNYAAALNVPGFHLHFITEDKTSGGHVLNLQF DNANLEISPIHEFDVQLPHTDDFAHSDLTQVTTSQVHQAESERK

**Example 2 - Heterologous expression of acetolactate decarboxylase, aldB**

The *aldB* gene from the strain *Brevibacillus brevis* encodes an acetolactate decarboxylases enzyme (ALDC) and was produced in *B. subtilis* using an integrated expression cassette consisting of the *B. subtilis aprE* promoter, the *B. subtilis aprE* signal peptide sequence, the mature aldB and a BPN' terminator. This cassette was cloned was cloned in the head to head orientation with respect to the expression cassette and the *aldB* expression cassette introduced into *B. subtilis* by homologous recombination.

A map of the vector containing the aldB gene (RIHI-aldB) is shown in Figure 2.

To produce aldB, a *B. subtilis* strain transformant containing *aldB* expression cassette was cultured in 15 ml Falcon tubes for 5 hours in TSB (broth) with 300 ppm beta-chloro-D-alanine (CDA), and 300 $\mu$l of this pre-culture was added to a 500 mL flask filled with 30 mL of cultivation media (described below) supplemented with 300 ppm CDA and 50$\mu$M $Zn^{2+}$. The flasks were incubated for 24, 48 and 72 hours at 33°C with constant rotational mixing at 180 rpm. Cultures were harvested by centrifugation at 14500 rpm for 20 minutes in conical tubes. The culture supernatants were used for protein determination and assays. The cultivation media was an enriched semi-defined media based on MOPs buffer, with urea as major nitrogen source, maltrin (maltodextrins and corn syrup solid) as the main carbon source.

**Example 3 - Protein Determination Methods**

Protein Determination by Stain Free Imager Criterion

Protein was quantified by SDS-PAGE gel and densitometry using Gel Doc™ EZ imaging system. Reagents used in the assay: Concentrated (2x) Laemmli Sample Buffer (Bio-Rad, Catalogue #161-0737); 26-well XT 4-12% Bis-Tris Gel (Bio-Rad, Catalogue #345-0125); protein markers "Precision Plus Protein Standards" (Bio-Rad, Catalogue #161- 0363); protein standard BSA (Thermo Scientific, Catalogue #23208) and SimplyBlue Safestain (Invitrogen, Catalogue #LC 6060. The assay was carried on as follow: In a 96well-PCR plate 50$\mu$l diluted enzyme sample were mixed with 50 $\mu$L sample buffer containing 2.7 mg DTT. The plate was sealed by Microseal 'B' Film from Bio-Rad and was placed into PCR machine to be heated to 70°C for 10 minutes. After that the chamber was filled by running buffer, gel cassette was set. Then 10 $\mu$L of each sample and standard (0.125-1.00 mg/ml BSA) was loaded on the gel and 5 $\mu$L of the markers were loaded. After that the electrophoresis was run at 200 V for 45 min. Following electrophoresis the gel was rinsed 3 times 5 min in water, then stained in Safestain overnight and finally destained in water. Then the gel was transferred to Imager. Image Lab software was used for calculation of intensity of each band. By knowing the protein amount of the standard sample, the calibration curve can be made. The amount of sample can be determined by the band intensity and calibration curve. The protein quantification method was employed to prepare samples of aldB acetolactate decarboxylases enzyme used for assays shown in subsequent Examples.

AldB sequence identification by MS - N and C-terminal amino acid determination

In preparation for sequence confirmation, an SDS-PAGE gel of isolated aldB truncation variants were analyzed by LC-MS/MS as described subsequently. In preparation for sequence confirmation, including N- and C-terminal determination, a protein band from an SDS-PAGE gel of aldB ferment sample was subjected to a series of chemical treatments. Between the individual steps the gel pieces were washed and shrunk using Milli-Q water, 50w/w% ethanol and absolute ethanol respectively. The protein was reduced/alkylated by DTT/Iodoacetamide treatment. A guanidination step was performed to convert lysines to homoarginines to protect lysine side chains from acetylation. The acetylation reaction using Sulfo-NHS-Acetate (Sulfosuccinimidyl Acetate) only modifies the protein N-terminal residue. The gel pieces were swelled with a buffer containing 40v/v% $^{18}O$ water:60v/v% $^{16}O$ water and the proteolytic enzymes used for protein digestion (Trypsin and $\alpha$-Chymotrypsin). The resulting peptides will contain mixtures of $^{18}O$ and $^{16}O$, except for the Carboxyl terminus which will retain the native $^{16}O$, as will be apparent from the isotopic pattern of the peptides. The peptide, originating from the protein N-terminus, will appear as the only acetylated peptide. After digestion the peptides were extracted from the gel pieces using 5w/w% formic acid and acetonitrile, then lyophilized and re-dissolved in 0.1w/w% TFA. The digestion products were separated (C18 column) and analyzed using a Proxeon nano-LC system followed by LTQ Orbitrap (Thermo Fisher) high resolution mass spectrometer and the amino acid sequence was deduced from the MS/MS fragment spectrum of the peptides, and the isotopic pattern of the peptides (using Xcalibur 2.0 SR2 software).

Based on this analysis, the N-terminus of the isolated full-length protein was confirmed to begin with A[25] (according to SEQ NO. 2) and the C-terminus of the isolated full-length protein was confirmed to end at position K[285] (according to SEQ NO. 2) by the acetylation and $^{18}$O-label methods as described above (see table 1). The N-terminus position A[25] at the mature aldB correspond with the predicted signal peptide cleavage determined by the Signal P 3.0 program (http://www.cbs.dtu.dk/services/SignalP/), set to SignalP-NN system, (Emanuelsson et al., (2007), Nature Protocols, 2: 953-971) of the gene transcript. Different N- and C-terminal truncation variants were further identified and their respective N- and C-terminus position according to SEQ NO. 2 are given in table 1.

*Table 1 Identified N- and C-terminus positions of aldB variants.*

|  | Position N- terminus | Position C-terminus |
|---|---|---|
| aldB full-length | A[25] | K[285] |
| aldB truncation variant 1 | Q[37] | K[285] |
| aldB truncation variant 2 | E[38] | K[285] |
| aldB truncation variant 3 | S[39] | K[285] |
| aldB truncation variant 4 | K[40] | K[285] |
| aldB truncation variant 5 | A[42] | K[285] |
| aldB truncation variant 6 | V[43] | K[285] |
| aldB truncation variant 7 | A[25] | S[275] |
| aldB truncation variant 8 | V[43] | S[275] |
| aldB truncation variant 9 | A[42] | S[275] |
| aldB truncation variant 10 | A[25] | Q[276] |
| aldB truncation variant 11 | V[43] | Q[276] |
| aldB truncation variant 12 | A[42] | Q[276] |

## Example 4 - Activity Assay Method

Spectrophotometric assay of α-acetolactate decarboxylase

α-Acetolactate decarboxylase (ALDC) catalyses the decarboxylation of α-acetolactate to acetoin. The reaction product acetoin can be quantified colourimetrically. Acetoin mixed with α-naphtol and creatine forms a characteristic red colour absorbing at OD522 nm. ALDC activity was calculated based on $OD_{522\,nm}$ and an acetoin calibration curve. The assay was carried out as follows: 20 mM acetolactate substrate was prepared by mixing 100 μL ethyl-2-acetoxy-2-methylacetoacetate (Sigma, Catalogue# 220396) with 3.6 ml 0.5 M NaOH at 10°C for 10 min. 20 ml 50 mM MES pH 6.0 was added, pH was adjusted to pH 6.0 and volume adjusted to 25 ml with 50 mM MES pH 6.0. 80 μL 20 mM acetolactate substrate was mixed with 20 μL enzyme sample diluted in 50 mM MES, pH 6.0, 0.6 M NaCl, 0.05% BRIJ 35 and 0.01% BSA. The substrate/enzyme mixture was incubated at 30°C for 10 min. Then 16 μL substrate/enzyme mixture was transferred to 200 μL 1 M NaOH, 1.0% α-naphtol (Sigma, Catalogue# 33420) and 0.1% creatine (Sigma, Catalogue# C3630). The substrate/enzyme/colour reagent mixture was incubated at 30°C for 20 min and then $OD_{522\,nm}$ was read. One unit of ALDC activity is defined as the amount of enzyme which produces 1 μmole acetoin per minute under the conditions of the assay.

## Example 5 - Expression analysis of aldB in various protease delete strains from B. subtilis

To identify suitable host for recombinant protein expression of the aldB gene in *B. subtilis,* three different strains with varying number of protease genes knocked-out were tested (2-delete, 5-delete and 7-delete). The term "knocked-out" as used herein refers to the inactivation of a gene such that there is no or decreased expression of the gene when compared to the parental strain in which the gene is not knocked out and/or there is no or decreased amount of the polypeptide encoded by the gene. A gene may be inactivated by, for example, deletion of the gene or part thereof or a regulatory element (such as a promoter) or by insertion of sequence into at least part of the gene or regulatory element (such as a promoter). The term "2-delete" as used herein refers to *B. subtilis* in which 2 protease genes have been knocked out. The term "5-delete" as used herein refers to *B. subtilis* in which 5 protease genes have been knocked out. The term "7-delete" as used herein refers to *B. subtilis* in which 7 protease genes have been knocked out. The "5-delete" strain as used herein was derived from the "2-delete" strain; thus, the 5-delete strain has 2 deleted protease genes which are the same as the genes deleted in the "2-delete" and 3 other deleted protease genes. The "7-delete" strain as used herein was derived from the "5-delete" strain; thus, the 7-delete strain has 5 deleted protease genes which are the same as the genes deleted in the "5-delete" and 2 other deleted protease genes (namely: vpr and wprA). Examples of protease genes which may be knocked out include genes which encode vpr, wprA, Epr, IspA, Bpr, NprE, AprE, ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD. The term "knocked-out" may be used interchangeably with the term "disrupted" or "deleted". The gene [according to SEQ NO. 4] was inserted in pBN-ssoU expression vector and samples were taken out over time as described in Example 1. Ferment samples were frozen in sample buffer to limit further potential degradation and all samples were collectively analysed by the Criterion SDS-PAGE analysis using Coomassie staining due to the absence of aromatic residues in aldB (see also example 3). The SDS-page of aldB expression in the 2-, 5- and 7-delete protease strain is shown in figure 3 with samples after 24, 48 and 72 hours of fermentation respectively. Bands identified either as full-length aldB (approximately 35kDa at the gel) or truncation variants hereof (28-35kDa at the gel) are marked on the gel. aldB variants were identified by MS sequencing as described in the method of example 3. Notable degradation was observed of aldB expression in the 2-delete protease strain with no full-length protein detectable during fermentation and truncation products present with the molecular size of 28 and 30kDa. AldB truncation products were also observed in the 5-delete strain, however with full-length protein present in the start of the fermentation (24 and 48 hrs) and truncation products being larger than when compared to truncation products from the 2-delete strain (32-35kDa at the gel). Conversion from the full-length protein to two major truncation products seems to progress during the fermentation in the 5-delete strain from 24 hrs to 72 hrs. Expression of aldB in the 7-delete protease strain only generated observable full-length aldB protein with no detectable truncation products throughout the fermentation.

ALDC activity was determined as described in (example 4) from fermentation samples and is shown in table 2. The activity was shown to peak after 48 hours where after it decreased at 72 hours in the 2- and 5-delete strain. Activity was increasing though-out the fermentation from aldB expression in the 7-delete strain. The 5-delete strain produced the highest activity after 72 hours (658 U/mL), followed by the 7-delete strain (538 U/mL) and the 2-delete strain (61 U/mL).

*Table 2 ALDC activity of aldB expressed in B. subtilis 2-, 5-, and 7-delete protease strains after various fermentation times.*

| | | ALDC activity [U/mL] | | |
| --- | --- | --- | --- | --- |
| | | **24 hrs** | **48 hrs** | **72 hrs** |
| **aldB** | **Protease 2-delete strain** | 110 | 112 | 61 |
| | | ALDC activity [U/mL] | | |
| | **Protease 5-delete strain** | 244 | 722 | 658 |
| | **Protease 7-delete strain** | 294 | 346 | 538 |

The concentration of aldB protein expressed was quantified using the Criterion software (Image Lab™ Software, BIO-RAD) with bovine serum albumin as standard and the concentration of aldB protein was calculated as sum of full-length and truncation products (shown in table 3). AldB protein increased until 48 hours of fermentation in the 2- and 5-delete, whereas it was increasing throughout fermentation in the 7-delete strain and highest after 72 hours of fermentation. After 72 hours of fermentation the aldB concentration was 0.32mg/mL in the 2-delete strain, 1.09mg/mL in the 5-delete strain and 0.68mg/mL in the 7-delete strain respectively.

*Table 3 Concentration of aldB protein expressed in B. subtilis 2-, 5-, and 7-delete protease strains after various fermentation times. Protein concentration was determined by Criterion SDS-PAGE analysis.*

| | | aldB protein concentration [mg/mL] | | |
|---|---|---|---|---|
| | | 24 hrs | 48 hrs | 72 hrs |
| aldB | Protease 2-delete strain | 0.18 | 0.32 | 0.32 |
| | Protease 5-delete strain | 0.29 | 1.02 | 1.09 |
| | Protease 7-delete strain | 0.25 | 0.43 | 0.68 |

The specific ALDC activity was calculated and is shown in table 4. The specific activity is decreasing over fermentation time for all strains. For all time points the observed specific activity was highest for the 7-delete strain compared to the 5- and 2-delete strain. After 72 hours of fermentation the specific ALDC activity of the 2-, 5- and 7-delete strain was 188, 602 and 796 U/mg.

*Table 4 Specific ALDC activity of aldB expressed in B. subtilis 2-, 5-, and 7-delete protease strains after various fermentation times.*

| | | Specific ALDC activity [U/mg] | | |
|---|---|---|---|---|
| | | 24 hrs | 48 hrs | 72 hrs |
| aldB | Protease 2-delete strain | 618 | 347 | 188 |
| | Protease 5-delete strain | 844 | 709 | 602 |
| | Protease 7-delete strain | 1157 | 799 | 796 |

The high specific activity ($\geq$800 U/mg) observed in material from the start of the 5-delete strain fermentation and the 7-delete strain correlate with relative abundance of the full-length protein in the sample (see table 4) and clearly suggest that the aldB truncation products constitute less efficient aldB enzymes variants. This suggest that the additional 2 proteases deleted in the 7-delete strain, extracellular serine protease (vpr) and cell wall protease (wprA), play an important role in the expression of the aldB enzyme.

*Table 5 The percentage of the full-length aldB protein out of the total aldB protein in sample, determined by Criterion SDS-PAGE analysis.*

| | | % full-length target protein | | |
|---|---|---|---|---|
| | | 24 hrs | 48 hrs | 72 hrs |
| aldB | Protease 2-delete strain | 0 | 0 | 0 |
| | Protease 5-delete strain | 50 | 20 | 0 |
| | Protease 7-delete strain | 100 | 100 | 100 |

**Example 6 - Protease spot plate assay of 2-, 5-and 7- delete Bacillus strains ferments**

The Principle of casein spot plate assay is to follow protease activity/hydrolysis by halo formation on casein plates.

Buffers: 100 mM McIlwaine, pH 6.0 (weigh 22.48 g $Na_2HPO_4.2H_2O$ and 7.74 g $C_6H_8O_7.H_2O$ and dissolve in 1000 ml dd$H_2O$) and 0,1 M Sodiumacetate buffer pH 5,60 (7,46 g Sodium acetate, anhydrous is dissolved in 800 ml dest. $H_2O$. pH is adjusted to 5,60 with acetic acid. It is filled to 1000 ml and pH adjusted if necessary).

Plates: Suspend 2% Casein and 2% Agarose (separately) in McIlwaine buffer and Heat both solutions in a microwave oven for approx. 5 min. Mix them together gently (when temp. is approx. 70 °C). Pour approx. 20-30 ml in Petri dishes and keep cool.

Small holes of 1 mm inside diameter were punched out of the gel and 5 $\mu$l of the enzyme solution was transferred to a hole and the plate was incubated at 40°C. The formation of haloes in the casein gel takes place as a function of time. A blank with buffer was added to one of the holes for comparison. As positive control the neutral protease from *B. subtilis* nprE (EC. 3.4.24.28) was used. 1 g nprE (Veron W) sample was diluted in 10 ml 0.1 M Sodium acetate buffer, pH 5.6 and mixed for 10 minutes. The sample was then filtered on Whatman GF/A and 5 $\mu$l of the nprE enzyme solution was transferred to the plate.

The result after 2 days of incubation at 40°C of the casein spot plate with aldB from the 2-, 5- and 7-delete strain is shown in figure 4. Clear halo formation is seen for the positive control and aldB from the 2-delete strain. A very faint and no halo formation are seen for aldB from the 5- and 7-delete strain respectively.

The result after 10 days of incubation show increased halo formation from the 2-delete strain, a small halo from the 5-delete strain and no halo from the 7-delete strain. This clearly suggests highest protease activity in the 2-delete strain ferment and decreasing in order with protease knock-out in the 5- and 7-delete strain ferments (as expected).

**Example 7 - Reduction in diacetyl and 2,3-pentanedione during beer fermentation by use of aldB**

The objective of this analysis was to test different acetolactate decarboxylase variants ability to reduce development of diacetyl and 2,3-pentanedione (Vicinal di-ketones, VDK) during a 7 days fermentation at 14°C.

Pure malt brew analysis

1100 g Munton's Light Malt Extract (Batch XB 35189, expiry date 24.05.2014) extract was dissolved in 3000 ml warm tapwater (45°C). This slurry was stirred for about 10 min until the liquid was homogeneous and the pH was adjusted to 5.2 with 2.5 M sulphuric acid. To the slurry was added 10 pellets of Bitter hops from Hopfenveredlung, St. Johann: Alpha content of 16,0 % (EBC 7.7 0 specific HPLC analysis, 01.10.2013), then split in 500mL blue-cap bottles and boiled for 1 hour to ensure protein precipitation and avoid potential microbial contamination. The final wort had an initial Specific Gravity of 1048 (i.e. 12 °Plato). 200g of the filtered wort were added to a 500 ml conical flask (Fermenting Vessel; FV), and then cooled 13°C. Each conical flask was dosed with 0.5% W34/70 (Weihenstephan) freshly produced yeast (1.0g yeast per 200g wort). The enzymes were dosed on similar ALDC activity (0.04 U/mL wort, 8 ALDC Units per 200g wort). The control fermentation with no enzyme received an amount of deionized water corresponding to the amount of enzyme sample.

The wort samples were fermented in 500 ml conical flasks under standardised laboratory test conditions at 14°C with gentle agitation at 150 rpm in an orbital incubator. When weight loss was less than 0.25g over 24 hours, fermentation temperature was decreased to 7°C. Fermentation was stopped after 8 days in total. 14 ml samples were taken out for diacetyl and 2,3-pentanedione analysis two times a day, preferably with 11 to 14 hours in between; at the end of fermentation only 1 sample per day was taken. Yeast was allowed to settle before take-out and each sample was cooled at 10°C for 10 minutes and then centrifuged at 4000rpm for 10 minutes at 8°C to sediment any residual yeast. The supernatant was separated from the yeast and samples for GC analysis were added 0.5 g NaCl per ml of sample. This slurry was transferred to a headspace vial and heat-treated at 65°C for 30 minutes before analysis for diacetyl and 2,3-pentanedione were carried out by gas chromatography with mass spectrometric detection (GCMS).

Analyses were carried out at an Agilent 6890N/5973N GC with CombiPAL headspace autosampler and MSChemStation acquisition and analysis software. The samples were equilibrated at 70°C for 10 minutes before 500μl of the gas phase above the sample was injected onto a J&W 122-0763 DB-1701column (60m x 0.25mmID x 1 μm). The injection temperature was 260°C and the system was operated with a constant helium flow of 2 ml/min. The oven temperature was: 50°C (2 min), 160°C (20°C/min), 220°C (40°C/min), hold 2 min. MS detection were made with 500 μL at a split ratio of 5:1 at selected ions. All sample were run in duplicates and standards were made using tap water with the addition of diacetyl or 2,3-pentanedione.

The concentration of a compound is calculated as

$$\text{Compound (mg/L)} = RF \ x \ \frac{Area}{1000 \ x \ W_s}$$

where,

RF      is the response factor of acetic acid
Area    is the GC-area of acetic acid
$W_s$     is the amount of sample used (in mL)

The limit of diacetyl quantification was determined to 0.016 mg/L and The limit of 2,3-pentanedione quantification to 0.012 mg/L.

To check that the addition of ALDC enzymes did not influence the Real Degree of Fermentation (RDF) and the produced alcohol by volume: RDF was measured using an Anton Paar (DMA 5000) following Standard Instruction Brewing, 23.8580-B28 and alcohol by Standard Instruction Brewing, 23.8580-B28.

Real degree of fermentation (RDF) value may be calculated according to the equation below:

$$RDF(\%) = \left(1 - \frac{RE}{{}^oP_{initial}}\right) \times 100$$

Where: RE = real extract = $(0.1808 \times {}^\circ P_{initial}) + (0.8192 \times {}^\circ P_{final})$, ${}^\circ P_{initial}$ is the specific gravity of the standardised worts before fermentation and ${}^\circ P_{final}$ is the specific gravity of the fermented worts expressed in degree plato.

In the present context, Real degree of fermentation (RDF) was determined from to the specific gravity and alcohol concentration.

Specific gravity and alcohol concentration was determined on the fermented samples using a Beer Alcolyzer Plus and a DMA 5000 Density meter (both from Anton Paar, Gratz, Austria). Based on these measurements, the real degree of fermentation (RDF) value was calculated according to the equation below:

$$RDF(\%) = \frac{OE - E(r)}{OE} \times 100$$

Where: E(r) is the real extract in degree Plato (°P) and OE is the original extract in °P.

The ability to reduce development of diacetyl and 2,3-pentanedione (Vicinal diketones, VDK) during a 7 days fermentation at 14°C was studied by addition of aldB from a *B. subtilis* 2-delete, 5-delete and 7-delete strain respectively. Three separate fermentations were conducted and VDK development analysed as described above. Fermentations with enzyme were always compared to a control without any enzyme added. For comparison the calculated VDK content (defined as the sum of diacetyl and 2,3 pentanedione) was normalized to the highest value obtained for the control sample without enzyme (as the absolute maximum was observed to vary from 1.48 to 2.76 mg VDK/L) and the results are shown in figure 5. All three aldB enzymes reduced the VDK development during fermentation compared to control. However, it is clear from examination of the peak VDK formed after approximately 40 hours that the enzymes perform different. A relative reduction in the peak VDK formed of 22.0, 25.6 and 62.7% was observed for the aldB from the 2-, 5- and 7-delete strain respectively. In addition, the fermentation time needed to get below the VDK level below the flavor threshold of diacetyl of 0.1 mg/mL, was observed to be approximately 138, 163 and 97 hours for the fermentation with aldB from the 2-, 5- and 7-delete strain respectively. Collectively, the ALDC performance in terms of VDK reduction during the beer fermentation seems highest for aldB produced in the 7-delete strain and less for aldB from 5- and 2-delete strains.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

The invention also relates to the following aspects as defined in the following numbered paragraphs:

1. A method for producing an acetolactate decarboxylase (ALDC) enzyme comprising:

A

i) providing a *Bacillus* host cell comprising a genetic alteration that causes said host cell to produce a decreased amount of an endogenous extracellular serine protease (vpr) and/or a cell wall protease (wprA) when compared to a parental cell, wherein said host cell is transformed with a nucleic acid encoding a heterologous ALDC enzyme in operable combination with a promoter; and

ii) cultivating said host cell under conditions suitable for the production of said heterologous ALDC enzyme, such that said heterologous ALDC enzyme is produced; or

B

i) providing a *Bacillus* host cell comprising a genetic alteration that causes the host cell to produce a decreased amount of an endogenous extracellular serine protease (vpr) and/or a cell wall protease (wprA) when compared to a parental cell, where the host cell is transformed with a nucleic acid that causes the host cell to overexpress an endogenous nucleic acid sequence encoding an ALDC enzyme when compared to the parental cell; and

ii) cultivating the host cell under conditions suitable for the production of ALDC enzyme, such that ALDC enzyme is produced.

2. The method of paragraph 1, further comprising recovering said produced ALDC enzyme.

3. The method of paragraph 1 or 2, wherein said *Bacillus* host cell is *B. subtilis.*

4. The method of any one of paragraphs 1 to 3, wherein said *Bacillus* host cell further lacks an endogenous minor extracellular serine protease enzyme (Epr).

5. The method of any one of paragraphs 1 to 4, wherein said *Bacillus* host cell further lacks an endogenous major intracellular serine protease enzyme (IspA), and/or an endogenous bacillopeptidase F enzyme (Bpr).

6. The method of any one of paragraphs 1 to 5, wherein said *Bacillus* host cell lacks a neutral metalloprotease enzyme (NprE).

7. The method of any one of paragraphs 1 to 6, wherein said host cell further lacks an endogenous serine alkaline protease enzyme (AprE).

8. The method of any one of paragraphs 1 to 7, wherein said host cell further lacks an endogenous minor extracellular serine protease enzyme (Vpr).

9. The method of any one of paragraphs 1 to 8, wherein said host cell further lacks an endogenous cell wall associated protease enzyme (WprA).

10. The method of any preceding paragraph, wherein the host further has decreased amounts of one or more additional proteases selected from the group consisting of ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD.

11. The method of any preceding paragraph, wherein the genetic alteration comprises a disruption of a gene present in the parental cell.

12. The method of paragraph 11, wherein said disruption is the result of deletion of all or part of the gene.

13. The method of paragraph 11, wherein disruption of the gene is the result of deletion of a portion of genomic DNA comprising the gene.

14. The method of any one of paragraphs 11-13, wherein disruption of the gene is the result of mutagenesis.

15. The method of any one of paragraphs 11-14, wherein disruption of the gene is performed using site-specific recombination.

16. The method of any one of paragraphs 11-15, wherein disruption of the gene is performed in combination with introducing a selectable marker at the genetic locus of the gene.

17. The method of any preceding paragraph, wherein the ALDC enzyme is from *Lactobacillus casei, Brevibacterium acetylicum, Lactococcus lactis, Leuconostoc lactis, Enterobacter aerogenes, Bacillus subtilis, Bacillus brevis, Lactococcus lactis DX,* or *Bacillus licheniformis.*

18. The method of any preceding paragraph, wherein the ALDC enzyme is from *Bacillus brevis* or *Bacillus licheniformis.*

19. The method of any preceding paragraph, wherein said ALDC enzyme has an amino acid sequence having at least 80% identity with any one selected from SEQ ID NO: 2, SEQ ID NO: 3, SEQ ID NO: 5, SEQ ID NO: 7, and SEQ ID NO: 8 or any functional fragment thereof.

20. A *Bacillus* host cell comprising a nucleic acid encoding a heterologous ALDC enzyme in operable combination with a promoter, wherein said host cell comprises a genetic alteration that causes said host cell to produce a decreased amount of an endogenous extracellular serine protease (vpr) and a cell wall protease (wprA); or a *Bacillus* host cell where the host cell comprises a genetic alteration that causes the host cell to produce a decreased amount of an endogenous extracellular serine protease (vpr) and/or a cell wall protease (wprA), and where the host cell comprises a nucleic acid that causes the host cell to overexpress an endogenous nucleic acid sequence encoding an ALDC enzyme when compared to the parental cell.

21. The *Bacillus* host cell of paragraph 20, wherein said *Bacillus* host cell is B. subtilis.

22. The *Bacillus* host cell of paragraph 20 or 21, wherein said host further has decreased amounts of an endogenous minor extracellular serine protease enzyme (Epr).

23. The *Bacillus* host cell of any one of paragraphs 20-22, wherein said host further has decreased amounts of an endogenous major intracellular serine protease enzyme (IspA), and/or an endogenous bacillopeptidase F enzyme (Bpr).

24. The *Bacillus* host cell of any one of paragraphs 20-23, wherein said host further has decreased amounts of a neutral metalloprotease enzyme (NprE).

25. The *Bacillus* host cell of any one of paragraphs 20 to 24, wherein said host cell further has decreased amounts of an endogenous serine alkaline protease enzyme (AprE).

26. The *Bacillus* host cell of any one of paragraphs 20 to 25, wherein said host cell further has decreased amounts of an endogenous minor extracellular serine protease enzyme (Vpr).

27. The *Bacillus* host cell of any one of paragraphs 20 to 26, wherein said host cell further has decreased amounts of an endogenous cell wall associated protease enzyme (WprA).

28. The *Bacillus* host cell of any one of paragraphs 20 to 27, wherein the host further has decreased amounts of one or more additional proteases selected from the group consisting of ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD.

29. The *Bacillus* host cell of any one of paragraphs 20 to 28, wherein the ALDC enzyme is from *Lactobacillus casei, Brevibacterium acetylicum, Lactococcus lactis, Leuconostoc lactis, Enterobacter aerogenes, Bacillus subtilis, Bacillus brevis, Lactococcus lactis DX,* or *Bacillus licheniformis.*

30. The *Bacillus* host cell of any one of paragraphs 20 to 29, wherein the ALDC enzyme is from *Bacillus brevis* or *Bacillus licheniformis.*

31. The *Bacillus* host cell of any one of paragraphs 20 to 30, wherein said ALDC enzyme has an amino acid sequence having at least 80% identity with any one selected from SEQ ID NO: 2, SEQ ID NO: 3, SEQ ID NO: 5, SEQ ID NO: 7, and SEQ ID NO: 8 or any functional fragment thereof.

32. A *Bacillus* host cell comprising a nucleic acid encoding a heterologous ALDC enzyme in operable combination with a promoter, wherein said host cell comprises a genetic alteration that causes said host cell to produce a decreased amount of an endogenous extracellular serine protease and/or a cell wall protease and/or a neutral metalloprotease capable of clipping a sequence from a C-terminus of said ALDC enzyme; or a *Bacillus* host cell where the host cell comprises a genetic alteration that causes the host cell to produce a decreased amount of an endogenous extracellular serine protease and/or a cell wall protease and/or a neutral metalloprotease capable of clipping a sequence from a C-terminus of the ALDC enzyme, and where the host cell comprises a nucleic acid that causes the host cell to overexpress an endogenous nucleic acid sequence encoding an ALDC enzyme when compared to the parental cell.

33. The host cell of paragraph 32, wherein said ALDC enzyme is *B. brevis* AldB and the protease is capable of clipping the sequence QVHQAESERK from said C-Terminus of said ALDC enzyme.

34. The *Bacillus* host cell of paragraph 32 or 33, wherein said *Bacillus* host cell is *B. subtilis.*

35. A *Bacillus* host cell comprising a nucleic acid encoding a heterologous ALDC enzyme in operable combination with a promoter, wherein said host cell comprises a genetic alteration that causes said host cell to produce a decreased amount of at least one protease when compared to the parental cell, wherein the protease is capable of cleaving a C-Terminus and/or N-Terminus of said ALDC enzyme; or a *Bacillus* host cell where the host cell comprises a genetic alteration that causes the host cell produce a decreased amount of a protease when compared to the parental cell, where the protease is capable of cleaving a C-Terminus and/or N-Terminus of the ALDC enzyme, and where the host cell comprises a nucleic acid that causes the host cell to overexpress an endogenous nucleic acid sequence encoding an ALDC enzyme when compared to the parental cell.

36. The *Bacillus* host cell of paragraph 35, wherein said *Bacillus* host cell is *B. subtilis.*

37. The *Bacillus* host cell of paragraph 35 or 36, wherein the protease is capable of cleaving at a corresponding C-terminus position 275 of SEQ ID No:5, SEQ ID No: 2 or SEQ ID NO 7.

38. The *Bacillus* host cell of paragraph 35 or 36, wherein the protease is capable of cleaving at a corresponding C-terminus position 276 of SEQ ID No:5, SEQ ID No: 2 or SEQ ID NO 7.

39. The *Bacillus* host cell of any one of paragraphs 35 to 38, wherein the protease is capable of cleaving at a corresponding N-terminus position 37 of SEQ ID No:5, SEQ ID No: 2 or SEQ ID NO 7.

40. The *Bacillus* host cell of any one of paragraphs 35 to 38, wherein the protease is capable of cleaving at a corresponding N-terminus position 38 of SEQ ID No:5, SEQ ID No: 2 or SEQ ID NO 7.

41. The *Bacillus* host cell of any one of paragraphs 35, 37 or 38, wherein the protease is capable of cleaving at a corresponding N-terminus position 39 of SEQ ID No:5, SEQ ID No: 2 or SEQ ID NO 7.

42. The *Bacillus* host cell of any one of paragraphs 35 to 38, wherein the protease is capable of cleaving at a corresponding N-terminus position 40 of SEQ ID No:5, SEQ ID No: 2 or SEQ ID NO 7.

43. The *Bacillus* host cell of any one of paragraphs 35 to 38, wherein the protease is capable of cleaving at a corresponding N-terminus position 42 of SEQ ID No:5, SEQ ID No: 2 or SEQ ID NO 7.

44. The *Bacillus* host cell of any one of paragraphs 35 to 38, wherein the protease is capable of cleaving at a corresponding N-terminus position 43 of SEQ ID No:5, SEQ ID No: 2 or SEQ ID NO 7.

45. The *Bacillus* host cell of any one of paragraphs 36 to 38, wherein the protease is capable of cleaving at a corresponding N-terminus position 39 of SEQ ID No:5, SEQ ID No: 2 or SEQ ID NO 7.

46. The *Bacillus* host cell of any one of paragraphs 35 to 45, wherein ALDC enzyme comprises an amino acid sequence that has at least 80% homology to SEQ ID No:5, SEQ ID No: 2 or SEQ ID NO 7.

47. The *Bacillus* host cell of any one of paragraphs 35 to 46, wherein said ALDC enzyme is *B. brevis* AldB and the protease is capable of cleaving the sequence QVHQAESERK from said C-Terminus of said ALDC enzyme.

48. The *Bacillus* host cell of any one of paragraphs 35 to 47, where the protease is a neutral metalloprotease.

49. The *Bacillus* host cell of any one of paragraphs 35 to 48, where the protease is thermolysin.

**Claims**

1. A method for producing an acetolactate decarboxylase (ALDC) enzyme comprising:

A

i) providing a *Bacillus* host cell comprising a genetic alteration that causes said host cell to produce a decreased amount of an endogenous extracellular serine protease (vpr) and/or a cell wall protease (wprA) when compared to a parental cell, wherein said host cell is transformed with a nucleic acid encoding a heterologous ALDC enzyme in operable combination with a promoter; and

ii) cultivating said host cell under conditions suitable for the production of said heterologous ALDC enzyme, such that said heterologous ALDC enzyme is produced;

wherein said ALDC enzyme has an amino acid sequence having at least 80% identity with any one selected from SEQ ID NO: 2, SEQ ID NO: 3, SEQ ID NO: 5, SEQ ID NO: 7, and SEQ ID NO: 8 or any functional fragment thereof having acetolactate decarboxylase activity;

wherein the genetic alteration comprises a disruption of a gene encoding said protease present in the parental cell, and

wherein said disruption is the result of deletion of all or part of the gene or wherein said disruption of the gene is the result of deletion of a portion of genomic DNA comprising the gene; or

B

i) providing a *Bacillus* host cell comprising a genetic alteration that causes the host cell to produce a decreased amount of an endogenous extracellular serine protease (vpr) and/or a cell wall protease (wprA) when compared to a parental cell, where the host cell is transformed with a nucleic acid that causes the host cell to overexpress an endogenous nucleic acid sequence encoding an ALDC enzyme when compared to the parental cell; and

ii) cultivating the host cell under conditions suitable for the production of ALDC enzyme, such that ALDC enzyme is produced;

wherein said ALDC enzyme has an amino acid sequence having at least 80% identity with any one selected from SEQ ID NO: 2, SEQ ID NO: 3, SEQ ID NO: 5, SEQ ID NO: 7, and SEQ ID NO: 8 or any functional fragment thereof having acetolactate decarboxylase activity;

wherein the genetic alteration comprises a disruption of a gene encoding said protease present in the parental cell, and

wherein said disruption is the result of deletion of all or part of the gene or wherein said disruption of the gene is the result of deletion of a portion of genomic DNA comprising the gene.

2.   The method of claim 1, further comprising recovering said produced ALDC enzyme.

3.   The method of Claim 1 or 2, wherein:

(a) said *Bacillus* host cell is *B. subtilis*; and/or
(b) said *Bacillus* host cell further lacks an endogenous minor extracellular serine protease enzyme (Epr); and/or
(c) said *Bacillus* host cell further lacks an endogenous major intracellular serine protease enzyme (IspA), and/or an endogenous bacillopeptidase F enzyme (Bpr); and/or
(d) said *Bacillus* host cell lacks a neutral metalloprotease enzyme (NprE); and/or
(e) wherein said host cell further lacks an endogenous serine alkaline protease enzyme (AprE); and/or
(f) said host cell further lacks an endogenous minor extracellular serine protease enzyme (Vpr); and/or
(g) said host cell further lacks an endogenous cell wall associated protease enzyme (WprA).
(h) the host further has decreased amounts of one or more additional proteases selected from the group consisting of ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD.

4.   The method of claim 1, wherein:

(a) disruption of the gene is performed using site-specific recombination; and/or
(b) disruption of the gene is performed in combination with introducing a selectable marker at the genetic locus of the gene.

5.   The method of any preceding claim, wherein the ALDC enzyme is from *Lactobacillus casei, Brevibacterium acetylicum, Lactococcus lactis, Leuconostoc lactis, Enterobacter aerogenes, Bacillus subtilis, Bacillus brevis, Lactococcus*

*lactis DX,* or *Bacillus licheniformis,* preferably wherein the ALDC enzyme is from *Bacillus brevis* or *Bacillus licheniformis.*

6. A *Bacillus* host cell comprising a nucleic acid encoding a heterologous ALDC enzyme in operable combination with a promoter,
   wherein said host cell comprises a genetic alteration that causes said host cell to produce a decreased amount of an endogenous extracellular serine protease (vpr) and/or a cell wall protease (wprA) and/or a neutral metalloprotease capable of clipping a sequence from a C-terminus of said ALDC enzyme; or
   a genetic alteration that causes the host cell to produce a decreased amount of an endogenous extracellular serine protease (vpr) and/or a cell wall protease (wprA) and/or a neutral metalloprotease capable of clipping a sequence from a C-terminus of the ALDC enzyme, and where the host cell comprises a nucleic acid that causes the host cell to overexpress an endogenous nucleic acid sequence encoding an ALDC enzyme when compared to the parental cell;
   further wherein said ALDC enzyme has an amino acid sequence having at least 80% identity with any one selected from SEQ ID NO: 2, SEQ ID NO: 3, SEQ ID NO: 5, SEQ ID NO: 7, and SEQ ID NO: 8, further wherein the genetic alteration comprises a disruption of a gene encoding said protease present in the parental cell and wherein said disruption is the result of deletion of all or part of the gene or wherein said disruption of the gene is the result of deletion of a portion of genomic DNA comprising the gene.

7. The *Bacillus* host cell of claim 6, comprising:
   a nucleic acid encoding a heterologous ALDC enzyme in operable combination with a promoter, wherein said host cell comprises a genetic alteration that causes said host cell to produce a decreased amount of an endogenous extracellular serine protease (vpr) and a cell wall protease (wprA);or a genetic alteration that causes the host cell to produce a decreased amount of an endogenous extracellular serine protease (vpr) and/or a cell wall protease (wprA), and where the host cell comprises a nucleic acid that causes the host cell to overexpress an endogenous nucleic acid sequence encoding an ALDC enzyme when compared to the parental cell.

8. The *Bacillus* host cell of Claim 7, wherein:

   (a) said *Bacillus* host cell is *B. subtilis*; and/or
   (b) said host further has decreased amounts of an endogenous minor extracellular serine protease enzyme (Epr); and/or
   (c) said host further has decreased amounts of an endogenous major intracellular serine protease enzyme (IspA), and/or an endogenous bacillopeptidase F enzyme (Bpr); and/or
   (d) wherein said host further has decreased amounts of a neutral metalloprotease enzyme (NprE); and/or
   (e) said host cell further has decreased amounts of an endogenous serine alkaline protease enzyme (AprE); and/or
   (f) the host further has decreased amounts of one or more additional proteases selected from the group consisting of ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD; and/or
   (g) the ALDC enzyme is from *Lactobacillus casei, Brevibacterium acetylicum, Lactococcus lactis, Leuconostoc lactis, Enterobacter aerogenes, Bacillus subtilis, Bacillus brevis, Lactococcus lactis DX,* or *Bacillus licheniformis*; and/or
   (h) the ALDC enzyme is from *Bacillus brevis* or *Bacillus licheniformis.*

9. The *Bacillus* host cell of claim 6, wherein said host cell comprises a genetic alteration that causes said host cell to produce a decreased amount of at least one neutral metalloprotease capable of cleaving a C-Terminus of said ALDC enzyme when compared to the parental cell.

10. The *Bacillus* host cell of Claim 9, wherein said *Bacillus* host cell is *B. subtilis.*

11. The *Bacillus* host cell of Claim 9 or 10, wherein: the protease is capable of cleaving at a corresponding C-terminus position 275 of SEQ ID No:5, SEQ ID No: 2 or SEQ ID NO 7; or
    the protease is capable of cleaving at a corresponding C-terminus position 276 of SEQ ID No:5, SEQ ID No: 2 or SEQ ID NO 7.

12. The *Bacillus* host cell of any one of claims 9 to 11, wherein ALDC enzyme comprises an amino acid sequence that has at least 80% homology to SEQ ID No:5, SEQ ID No: 2 or SEQ ID NO 7.

**13.** The *Bacillus* host cell of any one of claims 9 to 12, wherein:

(a) said ALDC enzyme is *B. brevis* AldB and the neutral metalloprotease is capable of cleaving the sequence QVHQAESERK from said C-Terminus of said ALDC enzyme; and/or
(b) the neutral metalloprotease is thermolysin.

*Eco* RI (7)

SSoU

*Eco* RI (384)

*Not* I (522)

aprE promoter region

*Spe* I (989)

aprE ss

aldB

*Nco* I (6334)

pBN-aldB
6844 bp

*Hin* dIII (1866)

Terminator

Neo

*Bam* HI (2119)

*Cla* I (2141)

*Apa* LI (2495)

bla

*Apa* LI (4239)

*Apa* LI (3741)

pMB1 origin

pBR322

*FIG. 1*

FIG. 2

*FIG. 3*

FIG. 4

**FIG. 5A**

**FIG. 5B**

*FIG. 5C*

**EUROPEAN SEARCH REPORT**

Europäisches Patentamt
European Patent Office
Office européen des brevets

Application Number

EP 20 19 5171

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 101 948 821 A (NANNING BIOCLONE BIOTECHNOLOGY CO LTD) 19 January 2011 (2011-01-19) * p. 1 li. 1-6, p. 2 li. 32-36, p. 3 3-p. 4 li. 10, p. 5 li. 1-7, p. 2 li. 2-4, p. 2 li. 17-30, p. 7 li. 3-4 * | 1-13 | INV. C12N9/88 C12N15/09 C12P1/04 |
| Y | XIAO-ZHOU ZHANG ET AL: "One-step production of lactate from cellulose as the sole carbon source without any other organic nutrient by recombinant cellulolytic Bacillus subtilis", METABOLIC ENGINEERING, vol. 13, no. 4, 1 July 2011 (2011-07-01), pages 364-372, XP055108491, ISSN: 1096-7176, DOI: 10.1016/j.ymben.2011.04.003 * table 2 * | 1-13 | |
| Y | WU SAU-CHING ET AL: "Functional production and characterization of a fibrin-specific single-chain antibody fragment from Bacillus subtilis: Effects of molecular chaperones and a wall-bound protease on antibody fragment production", APPLIED AND ENVIRONMENTAL MICROBIOLOGY, AMERICAN SOCIETY FOR MICROBIOLOGY, US, vol. 68, no. 7, 1 July 2002 (2002-07-01), pages 3261-3269, XP002519158, ISSN: 0099-2240, DOI: 10.1128/AEM.68.7.3261-3269.2002 * table 1 * | 1-13 | TECHNICAL FIELDS SEARCHED (IPC) C12N C12P |
| Y | WO 2009/062942 A2 (NOVOZYMES AS [DK]; KYHSE-ANDERSEN JAN [DK]) 22 May 2009 (2009-05-22) * p. 43 li. 8-11; example 3 * | 1-13 | |

-/--

The present search report has been drawn up for all claims

| Place of search | Date of completion of the search | Examiner |
|---|---|---|
| Munich | 11 March 2021 | Landré, Julien |

EPO FORM 1503 03.82 (P04C01)

page 1 of 2

Europäisches
Patentamt

European
Patent Office

Office européen
des brevets

# EUROPEAN SEARCH REPORT

Application Number

EP 20 19 5171

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 829 959 A1 (KAO CORP [JP]; ORGANIZATION OF SHINSHU UNIVER [JP]) 5 September 2007 (2007-09-05) * the whole document * ----- | 1-13 | |
| Y | WO 2009/022162 A1 (COBRA BIOLOG LTD [GB]; NEWCASTLE UNIVERSITY [GB]; CRANENBURGH ROCKY [G) 19 February 2009 (2009-02-19) * the whole document * ----- | 1-13 | |

TECHNICAL FIELDS
SEARCHED      (IPC)

The present search report has been drawn up for all claims

| Place of search | Date of completion of the search | Examiner |
|---|---|---|
| Munich | 11 March 2021 | Landré, Julien |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

.........................................................................
& : member of the same patent family, corresponding
  document

EPO FORM 1503 03.82 (P04C01)

page 2 of 2

## ANNEX TO THE EUROPEAN SEARCH REPORT
## ON EUROPEAN PATENT APPLICATION NO.

EP 20 19 5171

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2021

| Patent document cited in search report | | Publication date | Patent family member(s) | | Publication date |
|---|---|---|---|---|---|
| CN 101948821 | A | 19-01-2011 | NONE | | |
| WO 2009062942 | A2 | 22-05-2009 | AU | 2008322998 A1 | 22-05-2009 |
| | | | BR | PI0820156 A2 | 20-10-2015 |
| | | | CA | 2705334 A1 | 22-05-2009 |
| | | | CN | 101910194 A | 08-12-2010 |
| | | | DK | 2220107 T3 | 13-02-2017 |
| | | | EP | 2220107 A2 | 25-08-2010 |
| | | | ES | 2613029 T3 | 22-05-2017 |
| | | | HR | P20170069 T1 | 24-03-2017 |
| | | | HU | E032930 T2 | 28-11-2017 |
| | | | LT | 2220107 T | 10-04-2017 |
| | | | PL | 2220107 T3 | 31-05-2017 |
| | | | PT | 2220107 T | 08-02-2017 |
| | | | US | 2011207916 A1 | 25-08-2011 |
| | | | US | 2014081001 A1 | 20-03-2014 |
| | | | WO | 2009062942 A2 | 22-05-2009 |
| EP 1829959 | A1 | 05-09-2007 | CN | 101084302 A | 05-12-2007 |
| | | | DK | 1829959 T3 | 08-07-2013 |
| | | | EP | 1829959 A1 | 05-09-2007 |
| | | | JP | 4485341 B2 | 23-06-2010 |
| | | | JP | 2006174707 A | 06-07-2006 |
| | | | US | 2009081726 A1 | 26-03-2009 |
| | | | WO | 2006068148 A1 | 29-06-2006 |
| WO 2009022162 | A1 | 19-02-2009 | NONE | | |

EPO FORM P0459

For more details about this annex : see Official Journal of the European Patent Office, No. 12/82

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62165690 B
- US 62166616 B
- US 62168415 B
- DK 149335 B
- US 5874276 A

- US 5246853 A
- US 5475101 A
- WO 9206209 A
- WO 0214490 A

**Non-patent literature cited in the description**

- **SINGLETON et al.** DICTIONARY OF MICROBIOLOGY AND MOLECULAR BIOLOGY. John Wiley and Sons, 1994
- **HALE ; MARHAM.** THE HARPER COLLINS DICTIONARY OF BIOLOGY. Harper Perennial, 1991
- **DIDERICHSEN et al.** *J Bacteriol.,* 1990, vol. 172 (8), 4315
- **HONDEL et al.** More Gene Manipulations In Fungi. Academic Press, 1991, 396-428

- **CAMPBELL et al.** *Curr. Genet.,* 1989, vol. 16, 53-56
- **ANAGNOSTOPOULOS C. ; J. SPIZIZEN.** *J. Bacteriol.,* 1961, vol. 81, 741-746
- **EMANUELSSON et al.** *Nature Protocols,* 2007, vol. 2, 953-971
- **BABE et al.** *Biotechnol. Appl. Biochem.,* 1998, vol. 27, 117-124